# EUROPEAN PATENT SPECIFICATION

(11) **EP 3 013 963 B1**
(45) Date of publication and mention of the grant of the patent: **05.07.2017**
(21) Application number: 14733704.2
(22) Date of filing: 16.05.2014
(51) Int. Cl.: C12N 15/82, C07K 14/785

(54) **PRODUCTION OF HUMAN PULMONARY SURFACTANT PROTEIN B IN PLANTS**
HERSTELLUNG DES MENSCHLICHEN OBERFLÄCHENAKTIVEN LUNGENPROTEINS B IN PFLANZEN
PRODUCTION DE LA PROTÉINE TENSIOACTIVE PULMONAIRE HUMAINE B CHEZ LES PLANTES

(30) Priority: 24.06.2013 ZA 201304666
(43) Date of publication of application: 04.05.2016
(73) Proprietor: Azargen Biotechnologies (Pty) Ltd., Stellenbosch, 7600 (ZA)
(72) Inventor: VENTER, Mauritz, 7130 Somerset West (ZA); ZWIEGELAAR, Jacobus, Petrus, Western Cape 7600 Stellenbosch (ZA)
(74) Representative: Docherty, Robert Charles
(86) International application number: PCT/IB2014/061494
(87) International publication number: WO 2014/207585

(56) References cited:
- WO-A1-2008/000445
- WO-A2-03/020899
- US-A- 6 031 075
- CHIARA LICO ET AL: "The use of plants for the production of therapeutic human peptides", PLANT CELL REPORTS, vol. 31, no. 3, 1 March 2012 (2012-03-01), pages 439-451, XP055143984, ISSN: 0721-7714, DOI: 10.1007/s00299-011-1215-7
- GOERKE J ED - WATERHAM H R ET AL: "Pulmonary surfactant: functions and molecular composition", BIOCHIMICA ET BIOPHYSICA ACTA. MOLECULAR BASIS OF DISEASE, AMSTERDAM, NL, vol. 1408, no. 2-3, 19 November 1998 (1998-11-19), pages 79-89, XP004276795, ISSN: 0925-4439, DOI: 10.1016/S0925-4439(98)00060-X cited in the application
- DESAI P N ET AL: "Production of heterologous proteins in plants: Strategies for optimal expression", BIOTECHNOLOGY ADVANCES, ELSEVIER PUBLISHING, BARKING, GB, vol. 28, no. 4, 1 July 2010 (2010-07-01), pages 427-435, XP027048556, ISSN: 0734-9750 [retrieved on 2010-02-10]

## Description

### BACKGROUND OF THE INVENTION

This invention relates to a method for expression of a human pulmonary surfactant protein B (SP-B) in plants and to SP-B isoforms produced by the method. In particular a mature SP-B peptide, isoforms of mature SP-B, or analogs thereof.

Pulmonary surfactants are detergent-like compounds produced naturally in the lungs and consist of a macromolecular complex of lipids and proteins lining the epithelial surface of the lung. Pulmonary surfactants are produced by type II alveolar cells and are essential to i) lower surface tension and increase lung compliance during breathing, ii) interact with host microbial pathogens and iii) stimulate immune cells in the lungs **[Creuwels et al 1997; Goerke 1998].** Pulmonary surfactants are highly conserved among species **[Cockshutt and Possmayer 1992; Jobe 1993]** and comprise of approximately 90% lipids and 10% proteins **[Creuwels et al 1997; Wilson and Notter 2011; Blanco et al 2012].**

The genes encoding pulmonary surfactant proteins SP-A (most abundant protein), SP-B, SP-C and SP-D have been studied and deficiency of these proteins are primarily associated with a wide range of respiratory diseases **[Mallory 2001; Haataja and Hallman 2002; Whitsett and Weaver 2002; Yurdakök 2004; Clark H, Clark LS (2005].**

The surfactant proteins, SP-B and SP-C, are low molecular weight and extraordinarily lipophilic (compared to the hydrophilic, high molecular weight oligomers SP-A and SP-D) and are the most important proteins, strongly associated with surfactant lipids, to affect surface tension properties influencing pulmonary surfactant function **[Weaver and Conkright 2001; Parra et al 2011].** Although initially it was believed that all four surfactant proteins were important in facilitating the adsorption of phospholipids to the air-liquid interface of the alveoli to reduce surface tension, it is now known that only SP-B is essential for this function. In particular, deficiency of SP-B is directly associated with respiratory distress syndrome (RDS) that is one of the most important causes of morbidity and mortality in premature-born infants worldwide **[Creuwels et al 1997; Engle et al 2008].** RDS can be treated by surfactant replacement therapy (SRT) and SP-B has been identified as the most valuable protein of the two lipophilic proteins, SP-B and SP-C for SRT applications **[Wilson and Notter 2011].** Different animal (bovine/pig) and/or synthetic preparations are currently being used for SRT in clinical trials worldwide.

Newer versions of surfactants such as SURFAXIN® are synthetic preparations containing peptides mimicking the natural SP-B protein. SURFAXIN® (lucinactant) is the 5th FDA approved drug to treat RDS in the USA. The other four FDA approved surfactants are EXOSURF® (colfosceril palmitate), SURVANTA® (beractant), CUROSURF® (poractant alpha) and INFASURF® (calfactant) **[www.fda.gov].** EXOSURF® is a synthetic surfactant, but is no longer marketed and the other 3 surfactants are animal-derived. Although other delivery methods are being investigated, exogenous surfactant treatments are primarily administered by injection through a catheter directly in the lungs **[Halliday 2008; Guttentag and Foster 2011].**

In addition to treating RDS, clinical trials using exogenous surfactant have been conducted to treat meconium aspiration syndrome (MAS), congenital diaphragmatic hernia (CDH), bronchopulmonary dysplasia (BPD), genetic disorders of the surfactant system and bronchiolitis, brought on by respiratory syncytial virus (RSV) **[Guttentag and Foster 2011].** Other respiratory diseases that have also been targeted for treatment using exogenous surfactant include acute (or adult)-RDS, asthma, viral infections, chronic obstructive pulmonary disease (COPD) **[Lusuardi et al 1992; Creuwels et al 1997; Griese M 1999; Stevens and Sinkin 2007; Zuo et al 2008]** and other neonatal respiratory disorders **[Finer 2004].**

New developments in surfactant therapy fall in 3 categories: 1) new indications, 2) new delivery methods and 3) new surfactants **[Guttentag and Foster 2011].** Potential drawbacks associated with the current surfactant preparations for RDS as well as other respiratory diseases include: 1) low clinical efficacy, 2) limited product supply, 3) health risk of especially animal-derived products and 4) high cost of product **[Mingarro et al 2008; Blanco et al 2012, Ma and Ma 2012].** One of the major strategies highlighted to overcome the inhibition of lung surfactant is to optimize the lipid and surfactant protein content in exogenous preparations **[Blanco et al 2012].**

There have been numerous developments in synthetic surfactant preparations and/or modification of lipid/protein content for comparative and clinical evaluation and several of the new synthetic surfactants contain either shorter recombinant versions of the native protein or synthetic analogues that mimic the functional properties of the important hydrophobic SP-B and SP-C peptides **[Lukovic et al** 2006; **Almlen et al 2010; Seehase et al 2012; Jordan and Donn 2013].** Although these preparations may show promising clinical results compared to the current formulations **[Seehase et al 2012; Jordan and Donn 2013],** challenges regarding the synthesis of functional hydrophobic surfactant proteins, especially SP-B, still remain **[Zuo et al 2008].**

Due to the complexity regarding proper expression and activity of extraordinarily lipophilic surfactant proteins, synthetic surfactants comprising SP-B and/or SP-C analogues may be offered as an alternative to animal-derived natural surfactants, but will not necessarily be clinically superior, especially if SRT applications have been extended to other lung diseases such as Acute Respiratory Distress Syndrome (ARDS) **[Zuo et al 2008].**

This strongly suggests that there is a need for the development of other technologies to produce functional extraordinarily lipophilic surfactant proteins such as SP-B.

### SUMMARY OF THE INVENTION

According to a first aspect of the invention there is provided a method of producing a pulmonary surfactant protein-B (SP-B) mature peptide, or analog thereof, in a plant cell, the method comprising the steps of:
(i) providing a nucleic acid sequence consisting of a polynucleotide sequence encoding a SP-B mature peptide, or analog thereof;
(ii) introducing the nucleic acid sequence into an expression vector adapted to express a polypeptide in a plant cell;
(iii) introducing the expression vector of step (ii) into a plant cell;
(iv) expressing the SP-B mature peptide or analog thereof in the plant cell of step (iii); and
(v) harvesting the SP-B mature peptide, or or analog thereof from the plant cell, wherein the encoded SP-B mature peptide sequence is set forth in SEQ ID NO: 3, or is a variant sequence at least 90% identical to SEQ ID NO: 3 and wherein the variant sequence is capable of lowering surface tension of a lipid bilayer membrane on interaction with phospholipids

Preferably, the SP-B mature peptide is derived from a human gene.

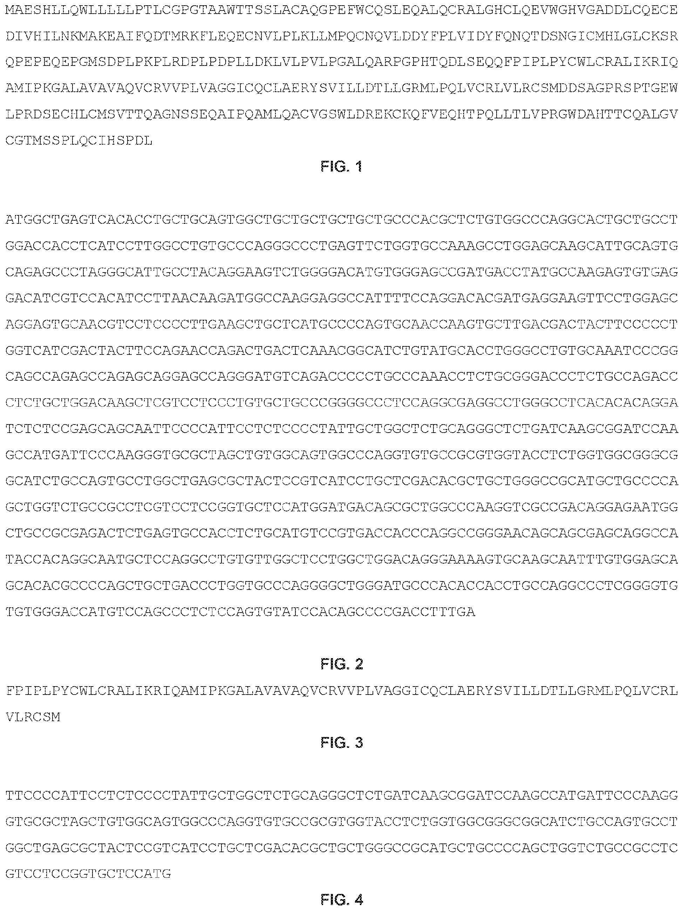

The polynucleotide sequence encoding the SP-B mature peptide, thereof may be:
(a) 100% identical to any one of SEQ I.D. NO 4 (Figure 4);
(b) a variant sequence at least 80% identical to any one of SEQ I.D. NOs 4 (Figure 4); or
(c) a sequence which hybridises under stringent conditions to the reverse complement of any one of SEQ I.D. NO 4 (Figures 4),
wherein the variant sequence or sequence which hybridises under stringent conditions to the reverse complement encodes a polypeptide capable of lowering surface tension of a lipid bilayer membrane on interaction with phospholipids.

Preferably, the number of hydrophobic amino acid residues encoded by the variant sequence or sequence which hybridises under stringent conditions to the reverse complement of any one of SEQ I.D. NO 4 is the same as, or greater than, the number of hydrophobic amino acid residues encoded by any one of SEQ I.D. NO 4 (Figure4).

More preferably, the polynucleotide sequence may have at least 85%, 90%, 95%, 97%, 98%, 99%, or 100% sequence identity to (b) above.

The SP-B mature peptide sequence may have 100% sequence identity to SEQ I.D. NO 3 (Figure 3), or may be a variant sequence at least 90 % identical to SEQ I.D. NO 3 (Figure 3), wherein the variant sequence is capable of lowering surface tension of a lipid bilayer membrane on interaction with phospholipids.

Preferably, the number of hydrophobic amino acid residues in the variant sequence is the same as, or greater than, the number of hydrophobic amino acid residues in SEQ I.D. NO 3 (Figure 3).

More preferably, the SP-B mature peptide sequence may have at least 95%, 97%, 98%, 99%, or 100% sequence identity to the peptide sequence set forth in SEQ ID NO: 3 (Figure 3).

The nucleic acid sequence may further comprise any one or more of the following elements:
(i) a polynucleotide sequence encoding a protease cleavage site, such as any one or more of an enterokinase, chymosin or Tobacco Etch Virus (TEV) protease cleavage site;
(ii) a polynucleotide sequence encoding a tag such as polyhistidine, Leptin, late embryogenesis abundant protein (LEA), Lectin, maltose binding protein (MBP) or glutathione S-transferase (GST), or other tags known to those skilled in the art; and
(iii) a polynucleotide sequence encoding a marker protein for detection, such as YPet, GFP, or others known to those skilled in the art.

Preferably, the one or more elements are combined as a fusion cassette together with the polynucleotide sequence encoding the SP-B mature peptide, or analog thereof.

The nucleic acid sequence may further comprise any one or more of the following elements:
(i) a plant promoter such as chrysanthemum RbcS1, 35S (such as CaMV35S), or others known to those skilled in the art, and a terminator sequence;
(ii) a polynucleotide sequence encoding a signal peptide such as the signal peptide region of equistatin or of *Nicotiana tabacum* thionin (NtSP); and
(iii) a polynucleotide sequence encoding a trafficking peptide such as an endoplasmic reticulum (ER)-trafficking peptide e.g., SEKDEL, KDEL, HDEL; an oil body-trafficking peptide (e.g. oleosin), a protein storage vacuole-trafficking peptide (e.g., a Vacuolar Sorting Determinant (VSD) from for example, barley lectin, common bean phaseolin, or soybean β-conglycinin α' subunit); a plastid, including a chloroplast, chromoplast or leucoplast-trafficking peptide (e.g., a peptide capable of interacting with the thylakoid membrane of a plastid such as the chloroplast targeting signal from the small subunit of Rubisco from Solanum); or another trafficking peptide known to those skilled in the art.

It is to be appreciated that polypeptide targeting to the cytoplasm is also encompassed within the scope of the invention, where there is omission of the inclusion of a tag. Furthermore, other targeting methods for plastids are encompassed within the scope of the invention, such as targeting of a transcript of the polypeptide of the invention to the chloroplast with the use of psbA regulatory 5'-UTR and 3' UTR regions in the transformation constructs.

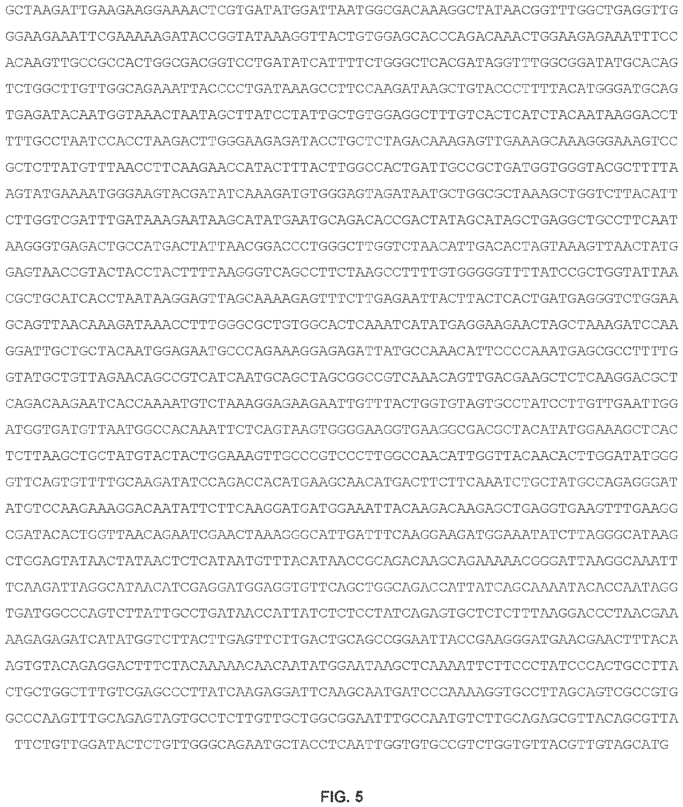

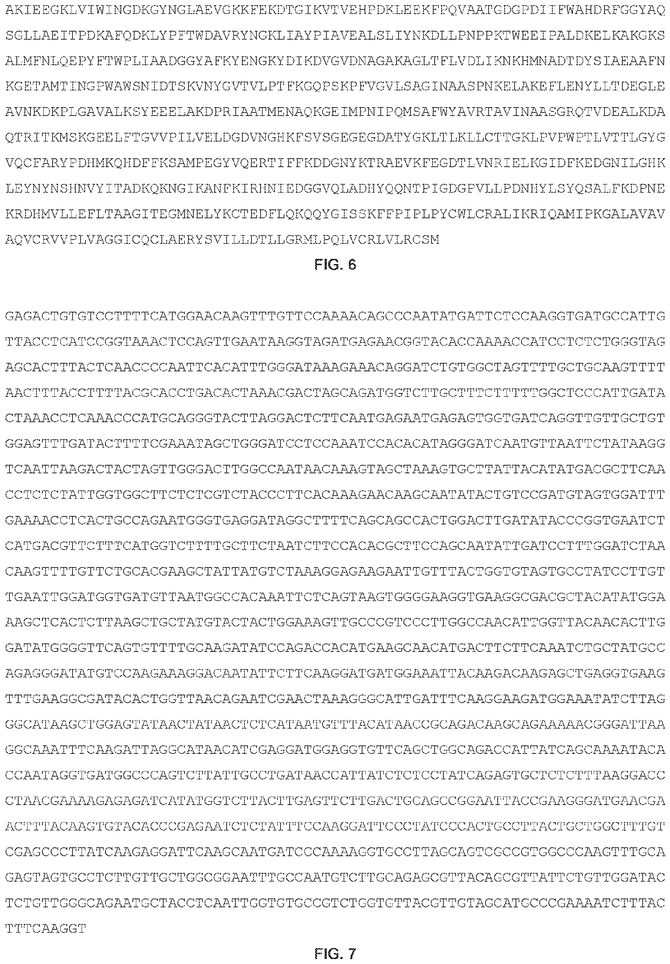

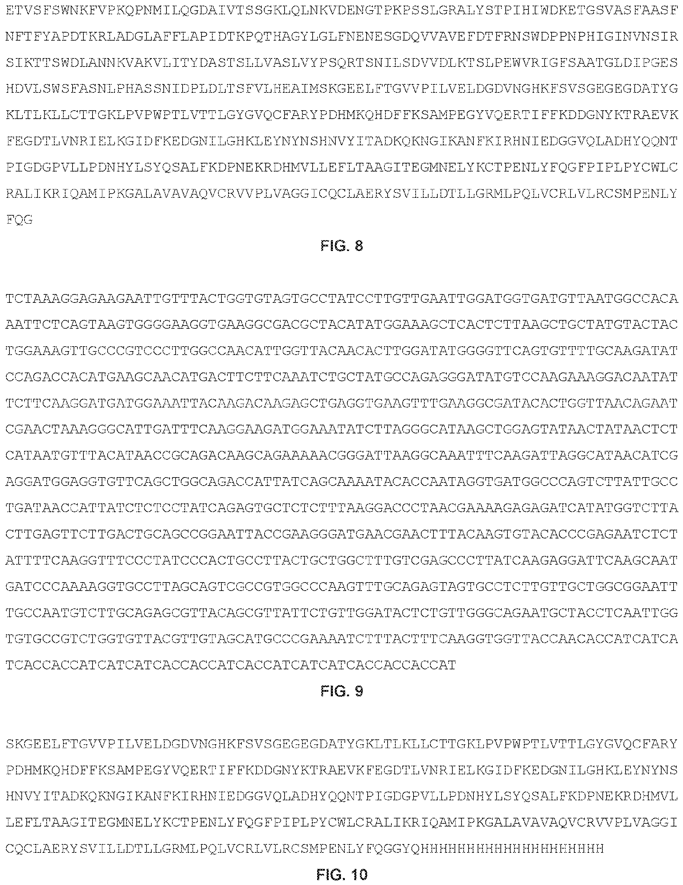

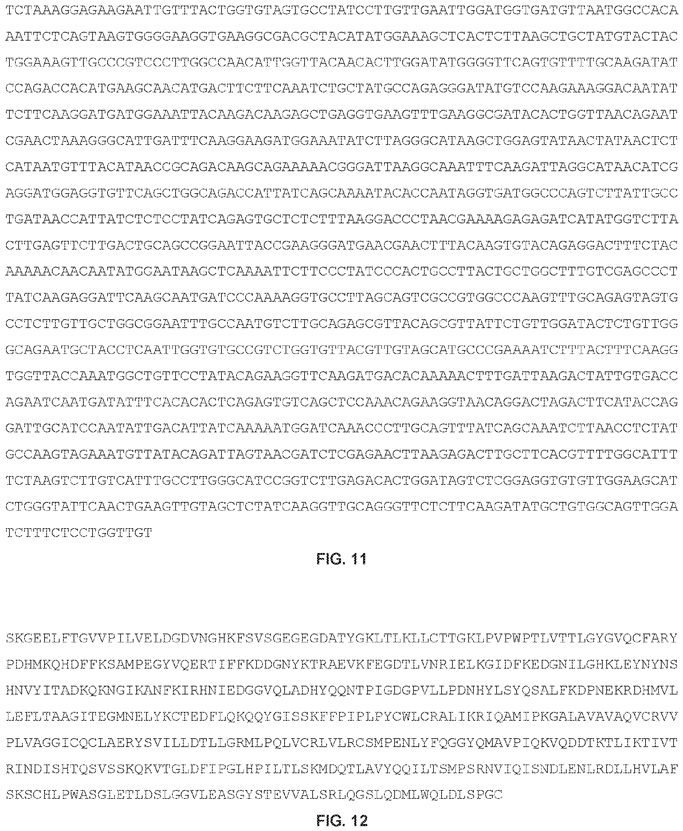

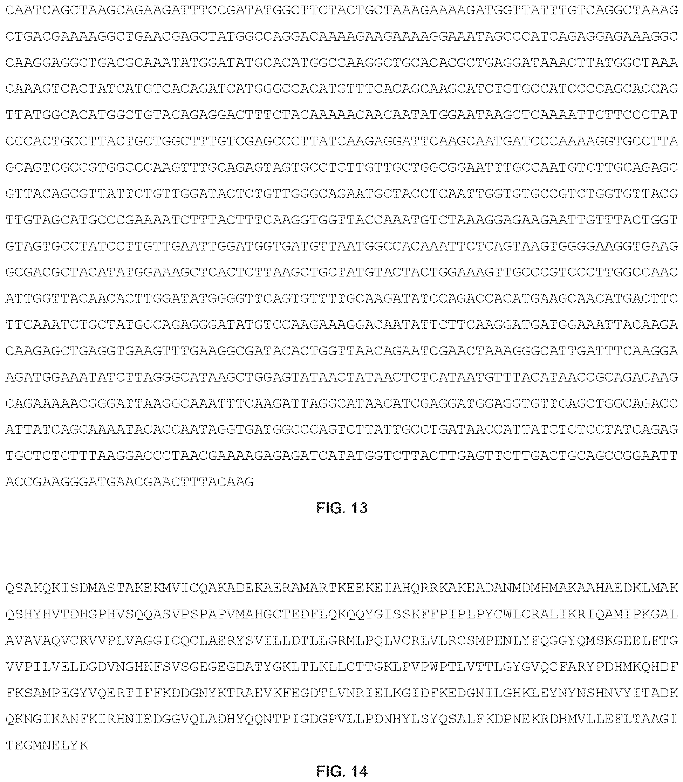

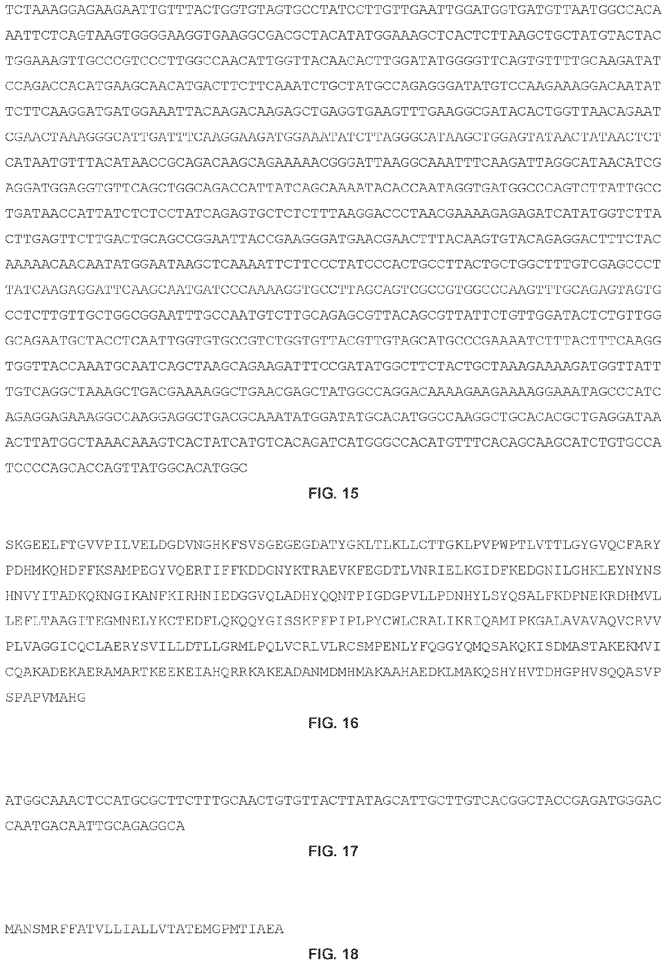

In particular, the fusion cassette may consist of any one of the following combinations in the order set out:
(i) MBP - YPet - chymosin - SP-B (for example, SEQ ID NO: 5; corresponding polypeptide sequence set out as SEQ ID NO: 6) (Figures 5 and 6);
(ii) Lectin - YPet - TEV - SP-B - TEV (for example, SEQ ID NO: 7; corresponding polypeptide sequence set out as SEQ ID NO: 8) (Figures 7 and 8);
(iii) YPet - TEV - SP-B - TEV - polyhistidine (for example, SEQ ID NO: 9; corresponding polypeptide sequence set out as SEQ ID NO: 10) (Figures 9 and 10);
(iv) YPet - chymosin - SP-B - TEV - Leptin (for example, SEQ ID NO: 11; corresponding polypeptide sequence set out as SEQ ID NO: 12) (Figures 11 and 12);
(v) LEA - chymosin - SP-B - TEV - YPet (for example, SEQ ID NO: 13; corresponding polypeptide sequence set out as SEQ ID NO: 14) (Figures 13 and 14); and
(vi) YPet - chymosin - SP-B - TEV - LEA (for example, SEQ ID NO: 15; corresponding polypeptide sequence set out as SEQ ID NO: 16) (Figures 15 and 16).

The fusion cassette may consist of or a polynucleotide sequence that is at least 80% identical to:
(a) any one of the polynucleotide sequences set out in SEQ ID NOs: 5, 7, 9, 11, 13, or 15; or
(b) a sequence which hybridises under stringent conditions to the reverse complement of any one of the the polynucleotide sequences set out in SEQ ID NOs: 5, 7, 9, 11, 13, or 15.

Further in particular, the nucleic acid sequence may comprise, in the order set out: a promoter sequence - signal peptide - any one of the fusion cassette combinations - trafficking peptide - terminator sequence.

For example, the nucleic acid sequence may comprise, in the order set out: RbcS1 promoter sequence - equistatin - any one of the fusion cassette combinations - KDEL - RbcS1 terminator sequence.

Optionally, the polynucleotide sequences may be codon optimised for expression in plant cells.

Optionally, the expression vector of step (ii) may further comprise a polynucleotide sequence encoding a suppressor protein adapted to inhibit post-transcriptional gene silencing in a plant cell. Alternatively, step (iii) may further include introducing into the plant cell a second plant vector comprising a polynucleotide sequence encoding a suppressor protein adapted to inhibit post-transcriptional gene silencing in the plant cell. For example, the suppressor protein may be the NSs protein of the tomato spotted wilt virus or the p19 of tomato bushy stunt virus, or others known to those skilled in the art.

Preferably, in step (iv) the expressed pulmonary surfactant protein-B pre-proprotein is retained in the ER of the plant cell.

Alternatively, in step (iv) the expressed pulmonary surfactant protein-B mature peptide is targeted to a plant component such as a plastid, oil body, protein storage vacuole or the cytoplasm of the plant cell.

The plant expression vector of step (ii) of the method may be an *Agrobacterium tumefaciens* vector.

Step (iii) of the method may comprise stable transformation of a plant cell by the introduction of the expression vector of step (ii) into the genetic material of the plant cell. For example, the method of introduction may be by agrobacterium transformation or agroinfiltration or other methods known to those skilled in the art.

The plant cell may be a plurality of plant cells in suspension culture, plant cells in tissue culture, plant cells in a leaf of a plant or a transgenic plant or any part thereof.

According to a further aspect of the invention, there is provided a polypeptide fusion cassette comprising a SP-B mature peptide, or analog thereof produced by the method of the invention wherein the encoded SP-B mature peptide sequence is set forth in SEQ ID NO: 3, or is a variant sequence at least 90% identical to SEQ ID NO: 3 and wherein the variant sequence is capable of lowering surface tension of a lipid bilayer membrane on interaction with phospholipids

According to a further aspect of the invention, there is provided a plant-based expression vector comprising the nucleic acid sequence according to the invention.

According to a further aspect of the invention, there is provided a plant cell comprising a plant-based expression vector or a SP-B mature peptide,or analog thereof produced by the method of the invention wherein the encoded SP-B mature peptide sequence is set forth in SEQ ID NO: 3, or is a variant sequence at least 90% identical to SEQ ID NO: 3 and wherein the variant sequence is capable of lowering surface tension of a lipid bilayer membrane on interaction with phospholipids

The disclosure provides a SP-B mature peptide, according to the invention for use in a method of preventing and/or treating a respiratory disease, condition or disorder in a subject.

The disclosure provides a use of the SP-B mature peptide, or analog thereof produced by the method of the invention for use in the manufacture of a medicament for use in a method of preventing and/or treating a respiratory disease, condition or disorder in a subject.

Preferably, the SP-B mature peptide is a human SP-B mature peptide.

The respiratory disease, condition or disorder may include any one or more of the following: respiratory distress syndrome (RDS) in infants, meconium aspiration syndrome (MAS), congenital diaphragmatic hernia (CDH), bronchopulmonary dysplasia (BPD), genetic disorders of the surfactant system and bronchiolitis, for example brought on by respiratory syncytial virus (RSV), acute (or adult)-RDS, asthma, viral infections, chronic obstructive pulmonary disease (COPD) and other neonatal respiratory disorders.

Preferably, the respiratory disease, condition or disorder is respiratory distress syndrome (RDS) in premature born infants.

The subject is preferably a human.

### BRIEF DESCRIPTION OF THE DRAWINGS

- **Figure 1**: shows the polypeptide sequence (381 aa) of the prepro-SP-B polypeptide (SEQ ID NO: 1);
- **Figure 2**: shows a cDNA sequence (1146 bp) encoding the prepro-SP-B polypeptide (SEQ ID NO: 2);
- **Figure 3**: shows the polypeptide sequence (79 aa) of the mature SP-B peptide (SEQ ID NO: 3);
- **Figure 4**: shows a cDNA sequence 237 bp) encoding the mature SP-B peptide (SEQ ID NO: 4);
- **Figure 5**: shows a cDNA sequence encoding the fusion cassette consisting of MBP - YPet - chymosin - SP-B (SEQ ID NO: 5);
- **Figure 6**: shows the polypeptide sequence of the the fusion cassette consisting of MBP - YPet - chymosin - SP-B (SEQ ID NO: 6);
- **Figure 7**: shows a cDNA sequence encoding the fusion cassette consisting of Lectin - YPet - TEV - SP-B - TEV (SEQ ID NO: 7);
- **Figure 8**: shows the polypeptide sequence of the the fusion cassette consisting of Lectin - YPet - TEV - SP-B - TEV (SEQ ID NO: 8);
- **Figure 9**: shows a cDNA sequence encoding the fusion cassette consisting of YPet - TEV - SP-B - TEV - polyhistidine (SEQ ID NO: 9);
- **Figure 10**: shows the polypeptide sequence of the the fusion cassette consisting of YPet - TEV - SP-B - TEV - polyhistidine (SEQ ID NO: 10);
- **Figure 11**: shows a cDNA sequence encoding the fusion cassette consisting of YPet - chymosin - SP-B - TEV - Leptin (SEQ ID NO: 11);
- **Figure 12**: shows the polypeptide sequence of the the fusion cassette consisting of YPet - chymosin - SP-B - TEV - Leptin (SEQ ID NO: 12);
- **Figure 13**: shows a cDNA sequence encoding the fusion cassette consisting of LEA - chymosin - SP-B - TEV - Ypet (SEQ ID NO: 13);
- **Figure 14**: shows the polypeptide sequence of the the fusion cassette consisting of LEA - chymosin - SP-B - TEV - Ypet (SEQ ID NO: 14);
- **Figure 15**: shows a cDNA sequence encoding the fusion cassette consisting of YPet - chymosin - SP-B - TEV - LEA (SEQ I D NO: 15);
- **Figure 16**: shows the polypeptide sequence of the the fusion cassette consisting of YPet - chymosin - SP-B - TEV - LEA (SEQ ID NO: 16);
- **Figure 17**: shows a cDNA sequence (93 bp) encoding the tobacco thionin like protein (NtSP) signal peptide (SEQ ID NO: 17);
- **Figure 18**: shows the polypeptide sequence (31 aa) of the tobacco thionin like protein (NtSP) signal peptide (SEQ ID NO: 18);
- **Figure 19**: shows a cDNA sequence encoding the equistatin signal peptide (SEQ ID NO: 19);
- **Figure 20**: shows the polypeptide sequence of the equistatin signal peptide (SEQ ID NO: 20);
- **Figure 21**: shows the pART27_preproSP-B_SEKDEL expression vector;
- **Figure 22**: shows the pART27-NtSP-His-mpSP-B expression vector;
- **Figure 23**: shows an embodiment of the nucleic acid sequence of the invention cloned into the plant expression vector, ImpactVector 1.3;
- **Figure 24**: shows RT-PCR analysis of **(A)** lane 1 - wild-type plant (non-transgenic) control; lanes 2 to 11 - plant lines 2, 7, 12, 18, 20, 22, 26, 38, 40 and 45 of the preproSP-B transcript, and **(B)** lanes 1 to 14 - plant lines 1, 6, 10, 13, 15, 17, 18, 22, 24, 25, 27, 31, 32 and 37 of the mpSP-B transcript. Top panels for both SP-B transcripts show positive control β-actin activity in respective plant lines;
- **Figure 25**: **A)** shows a representative western blot of human recombinant preproSB-B protein extracted from plant leaves: left panel - non-transgenic wild-type tobacco control; right panel - transgenic tobacco plant line 45. Protein samples volumes were from 25 µl down to 5 µl; **B)** shows a representative western blot of human recombinant mpSP-B and preproSP-B plant lines: lane C+ - SP-B positive control (Abnova); lane 2 - non-transgenic wild-type tobacco control; lane 3 - transgenic mpSP-B tobacco plant line 37; lanes 4 and 5 - transgenic preproSP-B tobacco plant lines 40 and 45;
- **Figure 26**: shows a representative western blot of Fc_3 with an expected size of 80.7 kDa extracted from plant leaves of transgenic plant lines. Lane M is protein standard (Life Technologies), lane wt = non-transgenic wild-type tobacco, lane C+ = purified YPet fluorescent protein positive control, lanes 1 to 9 = transgenic plant lines;
- **Figure 27**: shows a representative western blot of Fc_7 with an expected size of 67.4 kDa extracted from plant leaves of transgenic plant lines. Lane M is protein standard (Life Technologies), lane wt = non-transgenic wild-type tobacco, lane C+ = purified YPet fluorescent protein positive control, lanes 1 to 9 = transgenic plant lines;
- **Figure 28**: shows a representative western blot of Fc_9 with an expected size of 40.6 kDa extracted from plant leaves of transgenic plant lines. Lane M is protein standard (Life Technologies), lane wt = non-transgenic wild-type tobacco, lane C+ = purified YPet fluorescent protein positive control, lanes 1 to 9 = transgenic plant lines;
- **Figure 29**: shows a representative western blot of Fc_10 with an expected size of 50 kDa extracted from plant leaves of transgenic plant lines. Lane M is protein standard (Life Technologies), lane wt = non-transgenic wild-type tobacco, lane C+ = purified YPet fluorescent protein positive control, lanes 1 to 9 = transgenic plant lines;
- **Figure 30**: shows a representative western blot of Fc_12 with an expected size of 50 kDa extracted from plant leaves of transgenic plant lines. Lane M is protein standard (Life Technologies), lane wt = non-transgenic wild-type tobacco, lane C+ = purified YPet fluorescent protein positive control, lanes 1 to 9 = transgenic plant lines; and
- **Figure 31**: shows a representative western blot of Fc_13 with an expected size of 50 kDa extracted from plant leaves of transgenic plant lines. Lane M is protein standard (Life Technologies), lane wt = non-transgenic wild-type tobacco, lane C+ = purified YPet fluorescent protein positive control, lanes 1 to 9 = transgenic plant lines.

### DETAILED DESCRIPTION OF THE INVENTION

The current invention provides a method for expression of a human pulmonary surfactant B mature peptide (SP-B) in plants.

Generally, recombinant proteins are currently produced by bacterial, yeast, insect- and/or mammalian cell production platforms **[Drugmand et al 2012; Huang et al 2012; Kim et al 2012; Mattanovich et al. 2012]** of which bacterial (*E.coli*) and mammalian (usually Chinese Hamster Ovary_CHO) cells are the predominant systems used for commercial production. Expression of recombinant SP-C versions in *E.coli* have been reported **[Lukovic et al 2006],** but traditionally, mammalian cell systems, specifically CHO cells, have been preferred. Over the years these different production platforms have been optimized to achieve higher protein production levels and improved industrial scalability **[Berlec and Strukelj 2013].** However, these systems have a variety of important drawbacks that include partially active or non-functional products (especially in *E.coli* where post-translational modification is lacking), high cost of production and safety issues (i.e. animal pathogens, allergic responses).

Furthermore, a recent study at the University of Michigan was conducted to explore and compare the views of neonatologists and parents of newborns regarding the use of animal-derived medications. The study revealed that the majority (92%) of neonatologists were concerned about exposure of newborns to animal-derived pharmaceutical agents and 58% of the parents chose a non-animal derived version with safety as the prime concern.

Compared to current bacterial, yeast, insect and mammalian recombinant protein production systems, the use of plant cells to express human recombinant proteins may offer greater advantages with regard to 1) safety: no immunogenic responses or animal/bacterial-derived contamination, 2) complexity: plants are higher organisms with protein assembly mechanisms similar to humans, which can synthesize complex biopharmaceutical proteins (unlike bacterial fermentation systems that lack post-translational modification processes), 3) cost and scalability: plant-based systems allow for significantly lower facility and production costs (compared to mammalian cell-based systems) and production can easily be scaled up to accommodate increased demands.

A wide variety of plant-based recombinant biopharmaceutical compounds such as therapeutic proteins and vaccine antigens have been expressed using different plant-based expression hosts, platforms and tissues, but there are many factors, genetic and environmental, that influence successful plant-based recombinant protein production, and each system must be optimised and empirically tested for the specific protein to be produced, particularly for proteins that are highly lipophilic which have unique challenges associated with expression, correct folding and purification.

The three major plant-based production platforms are: 1) stable transformation, integration of foreign DNA in plant genome, 2) transient transformation, using viral vector and 3) plant cell culture. Each platform has a distinct advantage or disadvantage for a particular protein candidate, using a specific plant host, regarding production cost and time, scalability and regulatory compliance.

An important factor when determining whether a plant-based production platform is appropriate for production of a particular biopharmaceutical protein candidate, is the need to be able to express biopharmaceutical proteins at commercially viable levels. Even more importantly, to enter clinical development, plant-based production platforms must conform to good manufacturing practice (GMP) regulations.

SP-B and analogs thereof can be synthetically produced and function has been shown to be dependant on the interaction of the lipophilic protein with phospholipids to lower surface tension. Indeed, several of the new synthetic surfactants contain either shorter recombinant versions of the native protein or synthetic analogues that mimic the functional properties of the important hydrophobic SP-B and SP-C peptides **[Lukovic et al 2006; Almlen et al 2010; Seehase et al 2012; Jordan and Donn 2013].** It was found that the early SP-B truncated analogs that were similar in sequence to the C-terminal of native mature SP-B had similar biological function (when combined with phospholipids) to naturally occurring SP-B. It was therefore determined that activity is not primarily involved with SP-B peptide folding or conformation, but rather the presence of the necessary hydrophobic residues in the final sequence.

Compared to animal-derived and/or synthetic compounds comprising synthetic peptide analogues that only mimic the natural human surfactant protein, plant-based recombinant human surfactant proteins may address some of the drawbacks associated with current mammalian produced and synthetic surfactant preparations.

Of the two extraordinarily lipophilic surfactant proteins, SP-B plays a key role in the maturation of surfactant and is more active than SP-C concerning biophysical interaction with lipids and physiological function in lung surfactant. Addition of SP-B enhances phospholipid mixture activity and lowers surface tension to reverse neonatal respiratory failure **[Pryhuber 1998].**

The applicants thus sought to investigate whether a plant-based platform might potentially offer a means for the production of functional copies of the important exceptionally lipophilic surfactant protein SP-B. The SP-B protein is naturally processed in the lungs from a 381 amino acid, 40-kDa pre-proprotein (SEQ ID NO: 1; corresponding polynucleotide sequence set out as SEQ ID NO: 2) to a 79 amino acid, 8-kDa mature peptide (SEQ ID NO: 3; corresponding polynucleotide sequence set out as SEQ ID NO: 4) **[Pryhuber 1998].** The applicant was able to successfully demonstrate the expression of both SP-B protein isoforms in transformed transgenic plants. It is expected that the method would be similarly successful with analogs of the SP-B mature peptide isoforms.

A polynucleotide sequence encoding a peptide of interest (e.g., a SP-B mature peptide, or fusion cassette thereof), may be or contain, a polynucleotide sequence, having at least 90% sequence identity (e.g., at least 95%, 97%, 98%, 99%, or 100% sequence identity) to the polynucleotide sequences encoding the corresponding wild-type polypeptide thereof (i.e. as set forth in SEQ ID NOs: 4), with the proviso that the variant sequence is also capable of lowering surface tension of a lipid bilayer membrane on interaction with phospholipids. In particular, the number of hydrophobic residues in the variant sequence should be the same as, or greater than, the correspondingwild-type SP-B mature peptide thereof as it occurs naturally. In addition, the functional peptides of interest may have at least 90 % sequence identity (e.g., at least 95%, 97%, 98%, 99%, or 100% sequence identity) to the naturally occurring peptide sequences (i.e. as set forth in SEQ ID NOs: 3), with the proviso that the variant sequence is also capable of lowering surface tension of a lipid bilayer membrane on interaction with phospholipids. In particular, the number of hydrophobic residues in the variant sequence should be the same as, or greater than, the corresponding wild-type SP-B mature peptide, thereof as it occurs naturally. The means for determination of percent identity between a particular polynucleotide or amino acid sequence and the polynucleotide or amino acid sequence set forth for a polynucleotide or protein is well known to those skilled in the art may be be performed with multiple commercially available sequence analysis programes such as the BLAST programme and others known to those skilled in the art such as provided on the U.S. government's National Center for Biotechnology Information web site (www.ncbi.nlm.nih.gov).

The polynucleotide sequence identity compared to polynucleotide encoding the SP-B mature peptide may exist over a region of the sequence that is about 100, 150, 200 consecutive nucleotide residues in length. The amino acid identity compared to a SP-B mature peptide may exist over a region of the sequence that is about 50 consecutive amino acid residues in length.

It is further to be appreciated that a number of nucleic acids can encode a polypeptide having a particular amino acid sequence and such degeneracy of the genetic code is well known to the art. Such variations in degeneracy are included in the scope of the invention. For example, codons in the coding sequence for a given polypeptide can be modified such that optimal expression in a particular species (e.g., plants) is obtained, using appropriate codon bias tables for that species.

The scope of the invention further covers polynucleotide sequences which are able to hybridise under high stringency to a complement of SEQ I.D. NOs: 4 with the proviso that the variant sequence encodes a peptide that is capable of lowering surface tension of a lipid bilayer membrane on interaction with phospholipids. In particular, the number of hydrophobic residues in the peptide encoded by the variant sequence should be the same as, or greater than, the corresponding wild-type SP-B mature peptide as it occurs naturally. Hybridization conditions are well known to those skilled in the art. Highly stringent conditions are defined as equivalent to hybridization in 6X sodium chloride/sodium citrate (SSC) at 45°C, followed by a wash in 0.2 X SSC, 0.1 % SDS at 65°C.

It is also to be appreciated that any of the synthetic sequences or analogues of SP-B mature peptide presently produced, such as those described in Almlen *et al.,* 2010 (CWLCRALIKRIQAMIPKGGRMLPQLVCRLVLRCS, also known as Mini-B; Seehase *et al*., 2012 (CWLCRALIKRIQALIPKGGRLLPQLVCRLVLRCS, a synthetic SP-B analog), or Jordan and Donn 2013 could similarly be produced in plants using the method of the invention.

As previously indicated, functional activity of an SP-B mature peptide analog is not primarily involved with SP-B peptide folding or conformation, but rather the presence of hydrophobic residues in the final sequence. As previously indicated, functional activity of a SP-B mature peptide analog is primarily associated with the presence of hydrophobic residues in the final sequence. Functional analogs of the SP-B mature peptides of the invention may therefore be any mature SP-B peptide or any peptide analog thereof comprising hydrophobic amino acid residues, which, when they appropriately interact with specific phospholipids, will lower surface tension of a lipid bilayer membrane.

As unreliable transgene expression may be problematic when using conventional plant-based expression constructs for expression of a highly lipophilic, membrane bound protein, such as SP-B mature peptide, the applicant investigated whether it might be possible to improve the expression levels and purification efficiencies of SP-B mature peptide by fusion of theSP-B mature peptide to various heterologous polypeptides including protein tags and trafficking polypeptides in a fusion cassette. Such a fusion tag combination can comprise several types of tags for purification, such as affinity tags, for example, FLAG, polyhistidine, hemagluttanin (HA), glutathione-S-transferase (GST), or maltose-binding protein (MBP); tags for detection (such as yellow fluorescent protein (YPet), luciferase, green fluorescent protein (GFP), or chloramphenicol acetyl transferase (CAT); and tags for enhanced expression and stability (such as an expression protein partner tag, for example, Leptin, late embryogenesis abundant protein (LEA), Lectin, maltose binding protein (MBP) or glutathione S-transferase (GST).

Trafficking polypeptides may in particular include those for ER targeting such as KDEL, SEKDEL, or HDEL (with or without a signal peptide such as equistatin), or others known to those skilled in the art. However, tags for targeting to various other compartments such as oil bodies (e.g. recombinant polypeptide targeting to seeds of transgenic plants by fusion with oleosin), protein storage vacuoles (e.g., by fusion of a recombinant polypeptide with a Vacuolar Sorting Determinant (VSD) from for example, barley lectin, common bean phaseolin, or soybean β-conglycinin α' subunit), plastids, including chloroplasts, chromoplasts and leucoplasts (e.g. through fusion of a polypeptide with a peptide capable of interacting with the thylakoid membrane of a plastid such as the chloroplast targeting signal from the small subunit of Rubisco from *Solanum*) are well known to those skilled in the art and may also be used. It is to be appreciated that polypeptide targeting to the cytoplasm is also encompassed within the scope of the invention, where there is omission of the inclusion of a tag. Furthermore, other targeting methods for plastids are encompassed within the scope of the invention, such as targeting of a transcript of the polypeptide of the invention to the chloroplast with the use of *psbA* regulatory 5'-UTR and 3' UTR regions in the transformation constructs.

Furthermore, the applicant investigated whether inclusion of a protease cleavage site flanking the SP-B mature peptide within the fusion cassette for splicing the SP-B mature peptide from the fusion partners may enhance expression and purification of the SP-B mature peptide. All fusions were thus separated by linker peptide(s) containing a protease recognition site. The protease recognition site(s) included were selected from tobacco etch virus (TEV) or chymosin or both, although an enterokinase cleavage site may also be used.

Additionally, the applicant included in the plant expression vector a signal peptide, such as the signal peptide region of equistatin or of *Nicotiana tabacum* thionin (NtSP). The plant promoter used may be the chrysanthemum RbcS1, 35S (such as CaMV35S), or other plant promoters known to those skilled in the art, together with an appropriate terminator sequence such as the RbcS1 terminator, the Nos polyA or the CaMV35S polyA terminator sequence.

In particular, the applicant designed six different polypeptide fusion cassettes and was able to successfully demonstrate transgenic plant-based expression of the mature processed 8kDa SP-B polypeptide for expression analysis in *Nicotiana tobaccum* (tobacco) plants.

The invention will be described by way of the following examples which are not to be construed as limiting in any way the scope of the invention.

### EXAMPLES

### EXAMPLE 1

### 1. Isolation and cloning of the human prepro-SP-B cDNA into a plant transformation vector

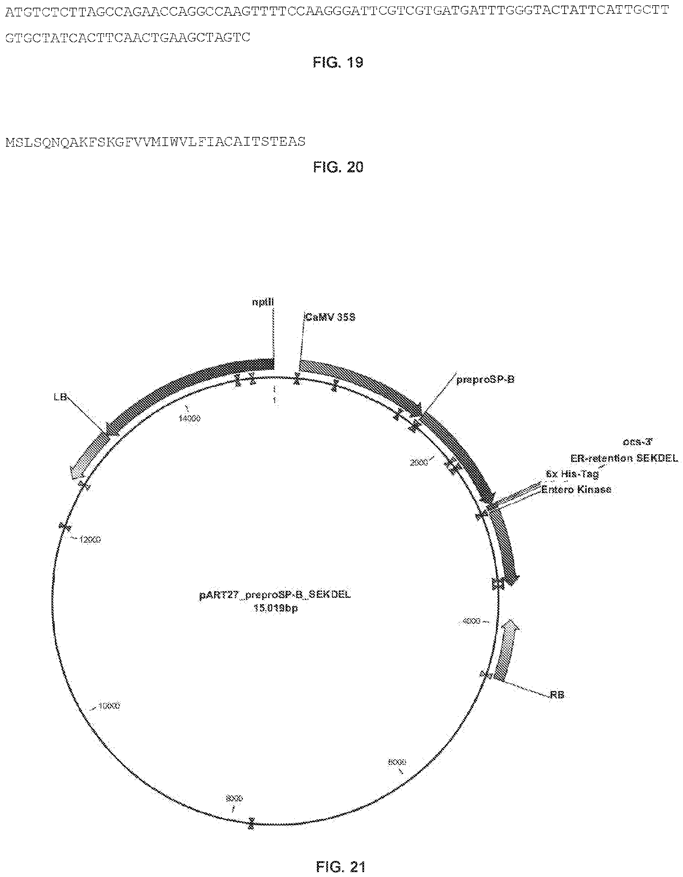

The human SP-B cDNA (1146 bp) encoding the prepro-SPB protein (GenBank acc NM_000542) (SEQ ID NO: 1; Fig. 1), was amplified from human lung cDNA (Ambion FirstChoice PCR-Ready) using the primers: preproSP-B_frw (5'- ATG GCT GAG TCA CAC CTG CTG-3') (SEQ ID NO: 21) and preproSP-B_rev (5'- TCA AAG GTC GGG GCT GTG GAT AC-3') (SEQ ID NO: 22). PCR amplification of prepro-SPB cDNA included a predenaturation at 94 °C for 30 sec followed by 35 cycles of amplification (94 °C denaturation, 30 sec; 50 °C annealing, 30 sec; 72 °C polymerization, 1 min 30 sec). PCR products were visualized in ethidium bromide-stained 1 % (w/v) agarose gels (results not shown), extracted and cloned in a pGEM®-T Easy Vector (Promega Corporation, Madison, USA). Selected clones were sequenced (ABI PRISM® 3100 genetic analyser) using the ready reaction kit with AmpliTaq® DNA polymerase (The Perkin Elmer Corporation, Norwalk, USA). A second prepro-SPB PCR fragment was generated using the primers: preproSP-B27_frw (5'-CCCCGAATTCATGGCTGAGTCACACCTGCTG-3') (SEQ ID NO: 23) incorporating a 5'-*Eco*RI restriction site and preproSP-B27_rev (5'-ggggAAGCTTTCAaagctcgtccttctcgctGTGATGGTGGTGGTGATGTTTGTCATCGT CATCAAGGTCCGGGCTGTGGATACACT -3') (SEQ ID NO: 24) incorporating an enterokinase site, His-Tag® sequence, a SEKDEL sequence for ER retention, TGA-stop codon and a *Hind*III restriction site at the 3'-end. PCR amplification using Supertherm Taq (Medox Biotech, India), included a predenaturation at 94 °C for 2 min followed by 35 cycles of amplification (94 °C denaturation, 30 sec; 50 °C annealing, 30 sec; 72 °C polymerization, 2 min). PCR product was visualized in ethidium bromide-stained 0.8 % (w/v) agarose gel, extracted and digested with *Eco*RI and *Hind*III restriction enzymes. Digested PCR product was cloned into pART7casette **[Gleave 1992]** prepared with *Eco*RI and *Hind*III followed by sub-cloning into the *Not*I sites of pART27 to yield pART27_preproSP-B_SEKDEL (see Fig. 21). Selected pART27 clones were sequenced (ABI PRISM® 3100 genetic analyser) using the ready reaction kit with AmpliTaq® DNA polymerase (The Perkin Elmer Corporation, Norwalk, USA) to confirm the preproSP-B_SEKDEL sequence.

### 2. Isolation and cloning of the mature SP-B peptide (mpSP-B) cDNA into the plant transformation vectors

The cDNA region (237 bp) encoding the mature SP-B peptide (SEQ ID NO: 3; Fig. 3) was amplified from human lung cDNA (Ambion FirstChoice PCR-Ready) using the primers: mpSP-B_frw (5'-GGCTCGAGTTCCCCATTCCTCTCCCCTA-3') (SEQ ID NO: 25) and mpSP-B_rev (5'-GGCAGATCTCATGGAGCACCGGAGGACGA-3') (SEQ ID NO: 26) generating *Xho*I and *Bgl*II sites at the 5'-and 3' ends respectively. PCR amplification using Elongase®enzyme mix, included predenaturation at 94 °C for 2 min followed by 35 cycles of amplification (94 °C denaturation, 30 sec; 60 °C annealing, 30 sec; 68 °C polymerization, 1 min). PCR products were visualized in ethidium bromide-stained 1 % (w/v) agarose gels (results not shown), extracted and cloned in a pGEM®-T Easy Vector (Promega Corporation, Madison, USA). Selected clones were sequenced (ABI PRISM® 3100 genetic analyser) using the ready reaction kit with AmpliTaq® DNA polymerase (The Perkin Elmer Corporation, Norwalk, USA).

A pET-14b vector (Novagen) containing an N-terminal His-Tag® sequence followed by a thrombin cleavage site was linearized at the *Xho*I restriction site. The mpSP-B fragments, digested with *Xho*I and *Bgl*II*,* were gel purified and resuspended in a ligation mix containing T4 DNA ligase, ligase buffer (Promega Corporation, Madison, USA) and linearized pET-14b vector to yield pET14B-mpSP-B. The ligation mixture was not transformed into competent cells, but rather was used as template for the construction of the downstream plant expression vectors.

A signal peptide region (SEQ ID NO: 18; Fig. 18) of the *Nicotiana tabacum* thionin (Genbank acc AB034956; cDNA (SEQ ID NO: 17; Fig. 17) was amplified from tobacco cDNA using the primers: NtSP-FRW (5'-GGCTCGAGATGGCAAACTCCATGCGCTTCTTT-3' (SEQ ID NO: 27) incorporating a *5'-Xho*I restriction site) and NtSP-REV (5'-CCAAGCTTTGCCTCTGCAATTGTCATTG-3' (SEQ ID NO: 28) to incorporate a *Hind*III restriction site at the 3'-end of the signal peptide). The tobacco thionin signal peptide targets expressed peptides to the apoplastic region in the plant cell. PCR amplification using Expand®enzyme mix, included predenaturation at 95 °C for 5 min followed by 35 cycles of amplification (95 °C denaturation, 30 sec; 55 °C annealing, 30 sec; 72 °C polymerization, 30 sec). PCR products were extracted and cloned in a pGEM®-T Easy Vector (Promega Corporation, Madison, USA) to yield pGEM-NtSP. Positive colonies were identified by restriction digest with *Xho*I and *Hind*III*.* The Nt-sp fragment was cloned into pART7casette **[Gleave 1992]** prepared with *Xho*I and *Hind*III and sub-cloned into the *Not*I sites of pART27 to yield to yield pART27_NtSP.

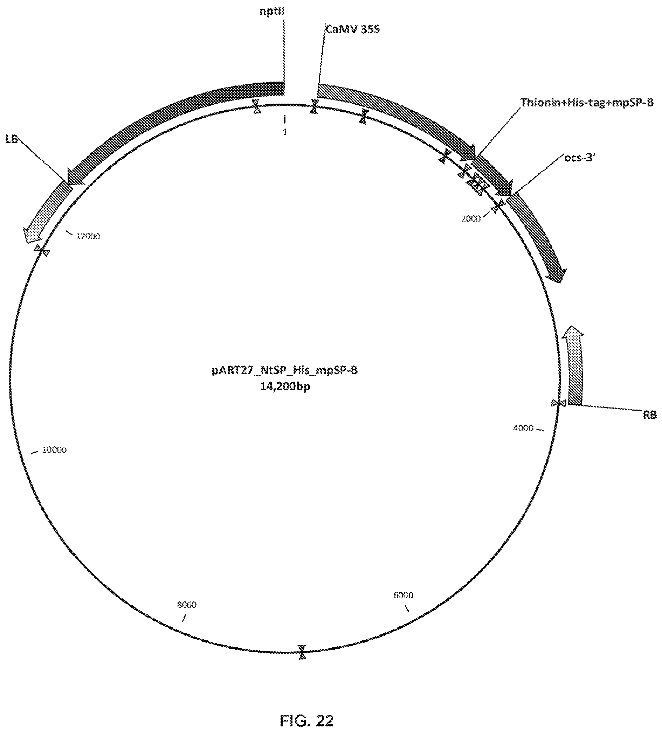

The His-Tag®-mpSP-B fusion was amplified including the 6x His- Tag®, but excluding the native 5'-ATG from pET14B-mpSP-B using the primers: NtSP27-FRW (5'- CCAAGCTTCATCATCATCATCATCACAG-3') (SEQ ID NO: 29) and NtSP27-REV (5'- TCTAGATCACATGGAGCACCGGAGGACGA-3') (SEQ ID NO: 30) generating *Hind*III and *Xba*I sites at the 5'-and 3' ends respectively. PCR amplification using Expand®enzyme mix and pET14B-mpSP-B ligation mix as template, included predenaturation at 95 °C for 5 min followed by 30 cycles of amplification (95 °C denaturation, 30 sec; 55 °C annealing, 30 sec; 72 °C polymerization, 30 sec). PCR products were extracted and cloned in a pGEM®-T Easy Vector (Promega Corporation, Madison, USA) to yield pGEM-His-NtSP-mpSP-B. Positive colonies were identified by restriction digest with *Hind*III and *Xba*I*,* excised from the agarose gel and cloned into *Hind*III/*Xba*I prepared pART27Nt-SP vector to yield pART27-NtSP-His-mpSP-B (see Fig. 22). Selected yield pART27-NtSP-His-mpSP-B clones were sequenced (ABI PRISM® 3100 genetic analyser) using the ready reaction kit with AmpliTaq® DNA polymerase (The Perkin Elmer Corporation, Norwalk, USA) to confirm NtSP-His-mpSP-B sequence.

### 3. Generation of transgenic plants

Tobacco plants were maintained on Murashige Skoog (MS) medium **[Murashige and Skoog 1962]** in a growth room regulated at a temperature of 22°C and a 16 h photoperiod. Expression cassettes, pART27_preproSP-B_SEKDEL and pART27-NtSP-His-mpSP-B, were electroporated into *Agrobacterium tumefaciens* strain LBA4404 **[Mattanovich et al 1989]** to generate recombinant *Agrobacterium tumefaciens.* Tobacco leaves (from plants not older than three months) were picked, washed, cut and transformed with the recombinant *Agrobacterium tumefaciens* using a standard leaf disc method **[Horsch et al 1985].** Plantlets were regenerated under kanamycin selection on MS medium and primary transgenic tobacco plantlets generated were hardened off and grown in a containment glasshouse under standard glasshouse conditions at 22 °C.

### 4. Molecular Analysis and RT-PCR of transgenic plant material

To evaluate transgenic status of tobacco plants, DNA was extracted from leaf material according to Sambrook *et al*., [1989] and purified using the Wizard® Genomic DNA Purification Kit (Promega Corporation, Madison, USA). PCR, using primers: SP-B_frw (5'-TCGAGTTCCCCATTCCTCTCCC-3') (SEQ ID NO: 31) and SP-B_rev (5'-AGACCAGCTGGGGCAGCATG-3') (SEQ ID NO: 32), consisted of a predenaturation cycle at 94 °C for 2 min followed by 35 cycles of amplification (94 °C denaturation, 30 sec; 55 °C annealing, 30 sec; 72 °C polymerization, 30 sec) to amplify a 211 bp fragment.

For RT-PCR analysis, total RNA was isolated using the RNeasy® Plant Mini Kit (Qiagen) according to manufacturer's instructions. RNA was treated with DNAse I using the NucleoSpin® RNA II kit according to manufacturer's protocol (MACHEREY-NAGEL). First strand cDNA synthesis and reverse transcriptase-PCR was carried out using the PrimeScript™ One Step RT-PCR Kit Ver.2 (Takara Bio) with the same primers used for transgenic analysis to isolate a 211 bp fragment from both preproSP-B and mpSP-B transcripts respectively. RT-PCR included an incubation reaction at 50 °C for 30 min and inactivation at 94 °C for 2 min followed by 35 cycles of amplification (94 °C denaturation, 30 sec; 60 °C annealing, 30 sec; 72 °C polymerization, 40 sec). PCR reactions were repeated with β-actin primers as 'housekeeping' control and PCR products were visualized in ethidium bromide-stained 1,2 % (w/v) agarose gels. All PCR reactions were carried out in a Perkin-Elmer GeneAmp® Thermocycler 9700 (Perkin Elmer Corporation, Wellesley, USA).

### 5. Protein purification, SDS-PAGE and western blot analysis

*Extraction method 1 (acetone and LEW buffer):* One gram plant tissue was washed 4X with 20 ml acetone to remove plant pigments. The tissue samples were allowed to dry after removal of the acetone. Five mL extraction buffer (LEW buffer from Protino Ni-TED kit- MACHEREY-NAGEL) + 1% triton and 30 mM - mercaptoethanol, was added and the proteins extracted for 2 hours on ice. The supernatant was collected by centrifugation at 12 000 rpm and the proteins precipitated with 50% ice cold acetone on ice for 2 hours. The precipitated protein was collected by centrifungation at 10 000 rpm and re-suspended in 500 mL extraction buffer supplemented with 100 mM DTT.

*Extraction method 2 (Methanol chloroform):* Plant tissue was extracted as described in US Patent Number 6,172,203 **[Hager and DePaoli, 2001]** with modifications. 2 Grams of plant leaf tissue ground up in liquid nitrogen and extracted with 40ml of 100% acetone to remove pigments, supernatant removed and acetone allowed to evaporate at 65 degrees C. Dried material was extracted with 10ml of Chloroform:Methanol (2:1) and supernatant collected. 5ml of 0.1M NaCl was used to extract the pellet and the supernatants combined. After centrifugation the organic phase was collected and back extracted with 5ml of NaCl and organic phase collected and precipitated with 6 volumes of acetone.

*Extraction method 3 (Phenol):* Plant tissue was extracted as described in **Wang et al., 2006** with or with the addition of 5% Beta Mercapthoethanol to all steps of the extraction.

*Chromogenic detection:* Proteins were separated on a 12.5% SDS-PAGE gel and electroblotted to PVDF membrane. The membrane was blocked with 5% skim milk in TBS-T for overnight followed by primary antibody (1:1000 dilution in 1% BSA in TBS-T) for 4 hours. SF-B was detected with secondary antibody diluted (1:10000) in TBS-T and the ECL kit from Amersham.

*Chemiluminescent detection:* Proteins were separated on a 8-16% Tris-Hepes SDS-PAGE gel and electroblotted to PVDF membrane. The membrane was blocked with Pierce Superblock for chemiluminescent detection for 16 hours followed by primary antibody (Abnova H00006439-B01P at a 1:20000 dilution in Superblock or Hycult HP9049 diluted 1:500 in superblock) for 1 hour. SF-B was detected with secondary antibody (Hycult HP1202 diluted 1:5000 in superblock) and Pierce Supersignal west Pico substrate according to the instructions of the manufacturer. Abnova H00006439-P01 and protein extracted from commercially obtained Curosurf^{®} surfactant was used as positive controls.

### 6. Peptide sequencing

Crude protein was excised from the SDS-PAGE gel and digested with trypsin. Mass spectrometry experiments were performed on a Nano-LC EASY-Column system connected to a LTQ Orbitrap Velos mass spectrometer (Thermo Fisher Scientific, Bremen, Germany).

### 7. Results

### 7.1 Transgenic and RT-PCR analysis

The applicants were able to demonstrate that the majority of transgenic plant lines, under kanamycin selection, confirmed presence of transgene (results not shown). Semi-quantitative RT-PCR analysis revealed transcriptional activity for cDNA's encoding both preproSP-B and mpSP-B protein isoforms respectively.

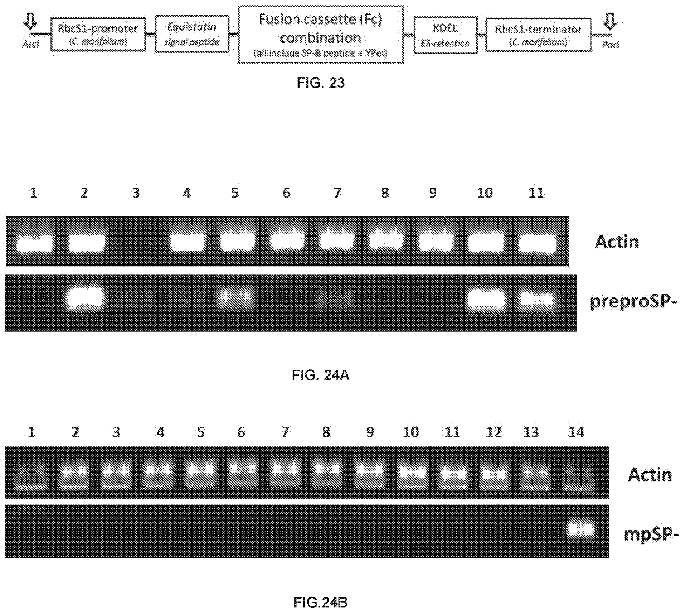

Seven preproSP-B lines and one mpSP-B line demonstrated activity on mRNA level (see Fig. 24A and B).

### 7.2. Western Blots

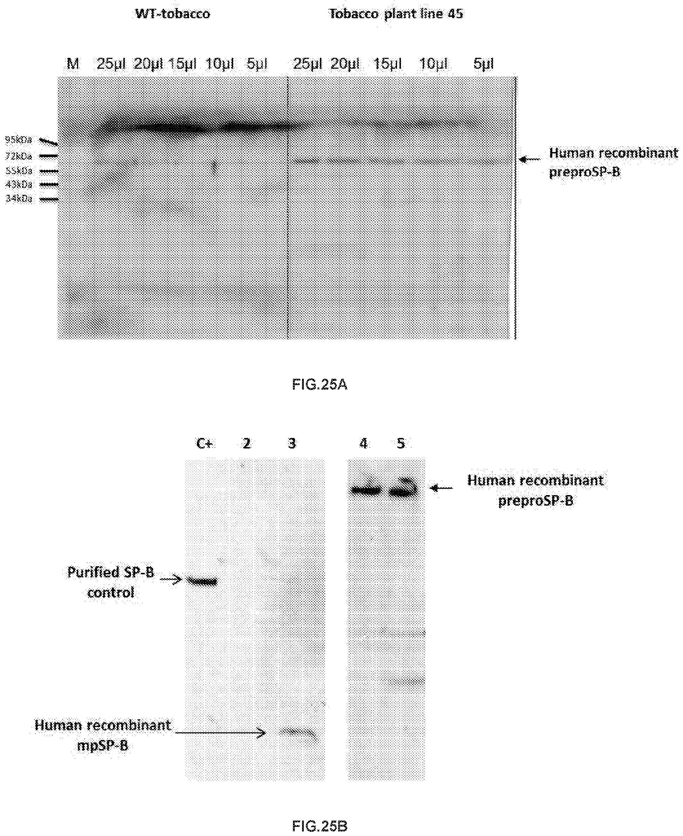

As illustrated in Fig. 25A, the western blot showed expression of human recombinant preproSP-B for the extraction method using acetone and LEW buffer.

Using the Phenol extraction method (see Fig. 25B), preproSP-B protein expression was demonstrated in preproSP-B plant line leaf tissue and mpSP-B protein expression in mpSP-B plant line leaf tissue respectively. The size of the preproSP-B protein bands detected, compared to purified SP-B control, indicates the possible formation of disulfide or other complexes.

Methanol:cloroform (2:1) extraction was also used to successfully extract mpSP-B protein from plant line leaf tissue (results not shown).

### 7.3 Peptide sequencing

Sequence analysis of crude protein extracts, excised from the SDS-PAGE gel and digested with trypsin revealed presence of a peptide YSVILLDTLLGR. Analysis of this peptide using UniProt database (www.uniprot.gov/blast) scored 100% identity to surfactant protein-B (results not shown).

### EXAMPLE 2

### 1. Materials and Methods

### 1.1 Fusion cassette (Fc design strategy, synthesis and cloning in plant transformation vector

All protein fusion cassettes comprised single or double copies of the human mature (8 kDa) SP-B peptide and the YPet fluorescent protein which is a yellow fluorescent protein (YFP) modified for Forster resonance energy transfer (FRET) applications **[Nguyen and Daugherty 2005].** Some fusion partners were chosen for their ability to be easily extracted via affinity chromatography while others were chosen for their solubility. Still others were chosen to enhance overall expression levels of the cassettes for easy detection in plants. The aim of the various fusion combinations was to find the optimal combination for easy detection and easy extraction in a water soluble form. All fusions were separated by linker peptide(s) containing a protease recognition site that would allow splicing of the fusion partners from the SP-B peptide. Recognition sites included in each cassette was either from tobacco etch virus (TEV), or chymosin, or both. Synthesis and subcloning of all fusion cassette sequence combinations were conducted by DNA2.0 (www.dna20.com). Fusion cassettes were synthesized and cloned into the *Nco*I/*Bgl*II sites of ImpactVector 1.3 (Plant Research International, Wageningen; (www.pri.wur.nl/UK/products/ImpactVector/). Within ImpactVector 1.3 each fusion expression cassette contains an N-terminal signal peptide from sea anemone (*Actinia equina*) equistatin, driven by the RbcS1 promoter of *Chrysanthemum morifolium* and a C-terminal KDEL sequence for endoplasmic reticulum (ER) retention and a terminator of rbcS1 gene from *C. morifolium* (see Fig. 22). Subsequently, each fusion expression cassette was excised using *Asc*I and *Pac*I restriction enzymes and re-cloned into the plant binary vector pCambia 1300 (CAMBIA, Canberra, Australia), of which the multiple cloning site was modified by DNA2.0 to accommodate *Asc*I and *Pac*I restriction and ligation.

### 1.2 Fusion cassette (Fc) designs from 5' to 3'

Six fusion cassettes were designed with various selected fusion partners based on the desired characteristics as set out above and then tested for expression in transgenic tobacco plant lines to determine whether the fusion cassettes would indeed result in successful expression of the fusion proteins of the expected size in transgenic tobacco plants.

### COMBINATION 1 (designated as Fc_3):

Maltose-binding protein (MBP)_Ypet_chymosin cleaving site_SP-B (Figs. 5 and 6). Fc_3 has an expected size of 80.73 kDa and contains a MBP fusion partner for enhanced solubility, correct folding and purification of target peptide.

### COMBINATION 2 (designated as Fc_7):

Lectin_YPet_TEV cleaving site_SP-B_TEV cleaving site (Figs. 7 and 8). Fc_7 has an expected size of 67.39 kDa and contains a soybean (*Glycine max*) lectin protein fusion partner to serve as an affinity tag for rapid purification.

### COMBINATION 3 (designated as Fc_9):

YPet_TEV cleaving site_SP-B_TEV cleaving site_poly-Histidine-tag (Figs. 9 and 10). Fc_9 has an expected size of 40.6 kDa and contains a polyhistidine-tag of twenty (20) histidine repeats for rapid and relative inexpensive purification of the target peptide via metal affinity chromatography.

### COMBINATION 4 (designated as Fc_10):

YPet_chymosin cleaving site_SP-B_TEV cleaving site_Leptin (Figs. 11 and 12). Fc_10 has an expected size of 55.89 kDa and contains a human leptin protein which is a product of the *obese* gene for enhanced target peptide stability and expression level.

### COMBINATION 5 (designated as Fc_12):

Late Embryogenesis Abundant (LEA) protein_chymosin cleaving site_SP-B_TEV cleaving site_YPet (Figs. 13 and 14). Fc_12 has an expected size of 50.91 kDa and contains an *Arabidopsis thaliana* LEA protein fusion partner for improved stability of the expressed protein and enhanced expression levels in response to water and temperature stress.

### COMBINATION 6 (designated as Fc_13):

YPet_chymosin cleaving site_SP-B_TEV cleaving site_ Late Embryogenesis Abundant (LEA) protein (Figs. 15 and 16). Fc_13 has the same fusion partners as Fc_12 but with a C-terminal LEA protein.

### 1.3 Generation of transgenic plants and genomic PCR

Tobacco plants (*Nicotiana tabacum* var Samsun) were maintained on MS medium **[Murashige and Skoog 1962]** in a temperature (22 °C) regulated growth room at a 16 h photoperiod. The pCambia expression vectors comprising fusion cassettes Fc_3, 7, 9, 10, 12 and 13 were introduced into *Agrobacterium tumefaciens* strain LBA4404 via electroporation **[Mattanovich et al 1989].** Tobacco leaves (from young plants) were transformed using a standard leaf disc method **[Horsch et al 1985].** Tobacco plantlets were regenerated under kanamycin selection (150 µg/mL) on MS medium and primary transgenic plantlets were hardened off and grown in a containment glasshouse under standard glasshouse conditions at 22 °C. To evaluate transgenic status of tobacco plants, genomic DNA was extracted from leaf material using the GeneJET Genomic DNA Purification Kit (Thermo Fisher Scientific Inc, USA). PCR, using primers: YPet_frw (5'-CTCAGTAAGTGGGGAAGGTGAAGGC-3') (SEQ ID NO: 33) and YPet_rev (5'-TGCCAGCTGAACACCTCCATCCTCG-3') (SEQ ID NO: 34), consisted of a predenaturation cycle at 94 °C for 2 min followed by 35 cycles of amplification (94 °C denaturation, 30 sec; 55 °C annealing, 30 sec; 72 °C polymerization, 30 sec) to amplify a 457 bp fragment using GoTaq® DNA Polymerase (Promega Corporation, Madison, USA). All PCR reactions were carried out in a Perkin-Elmer GeneAmp® Thermocycler 9700 (Perkin Elmer Corporation, Wellesley, USA) and PCR products were visualized in ethidium bromide-stained 1,2 % (w/v) agarose gels.

### 1.4 Protein extraction and Western blot analysis

Proteins were extracted from the plant tissue with a buffer containing 1 x phosphate buffered saline (PBS, Sigma-Aldrich, 79383) with 7M Urea, 2M Thiourea, 5% CHAPS and 5% Beta Mercaptho Ethanol. 500mg of plant leaf material that was ground up in liquid Nitrogen was extracted with 1ml of extraction buffer. The extract was vortexed for 1 min and left on the bench for 10 minutes where after it was vortexed again and centrifuged for 10 min at 13000 x g. The supernatant was used directly to load into the gel for western blot analysis. SDS PAGE was performed with the Life technologies Bolt™ system (Life Technologies) using Bis-Tris MOPS buffer and 4-12% gradient gel. Transfer and western blot was performed with the Life technologies iBlot® system according to the instructions of the manufacturer. Chromogenic detection was done using the iBlot® chromogenic western detection kit (Life Technologies, IB7410-01). All transgenic plant lines from each fusion protein cassette were screened with an antibody that specifically recognises GFP mutants (Thermo Fischer Scientific, PA1-28521). To serve as a positive control, fluorescent protein (YPet) was extracted from transgenic plants transformed with pCambia-ImpactVector 1.1 of which RbcS1 promoter and equistatin signal peptide was replaced with double enhancer CaMV 35S promoter only expressing YPet.

### 1.5 Isolation and purification of SF-B peptide

His-tag purified sample in solution from construct Fc_9 (GFP-His-tag fusion cassette) was prepared for N-terminal peptide sequencing. Protein extract was digested with trypsin. Smaller peptide sequences were generated and compared to protein sequences in international databases (UniPROT and SwissPROT).

### 2. Results

### 2.1 Western blot analysis

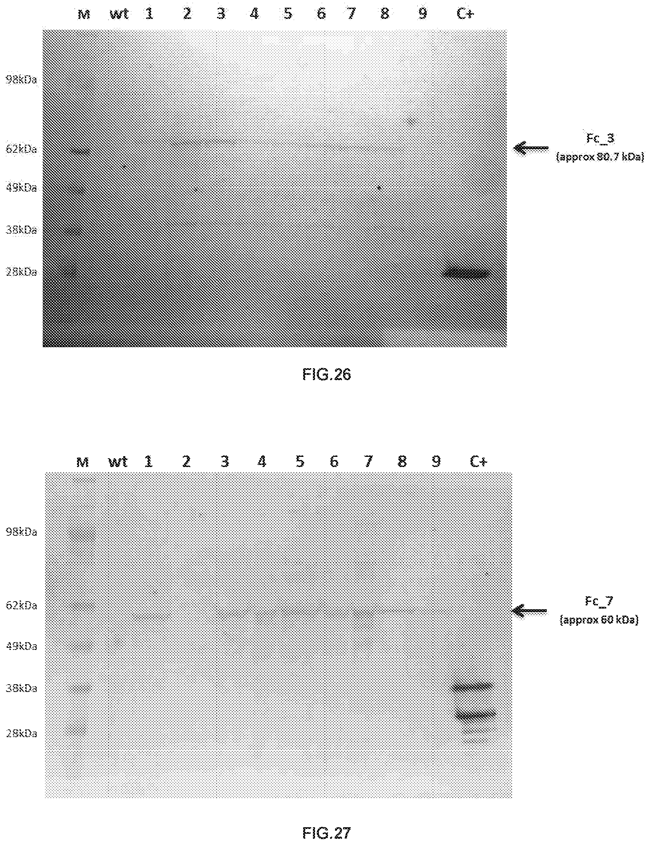

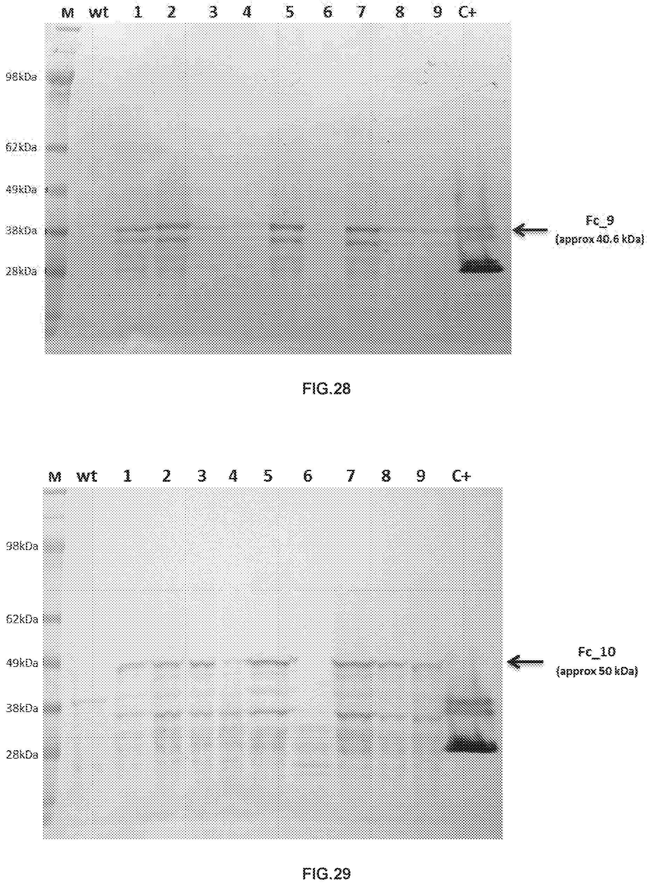

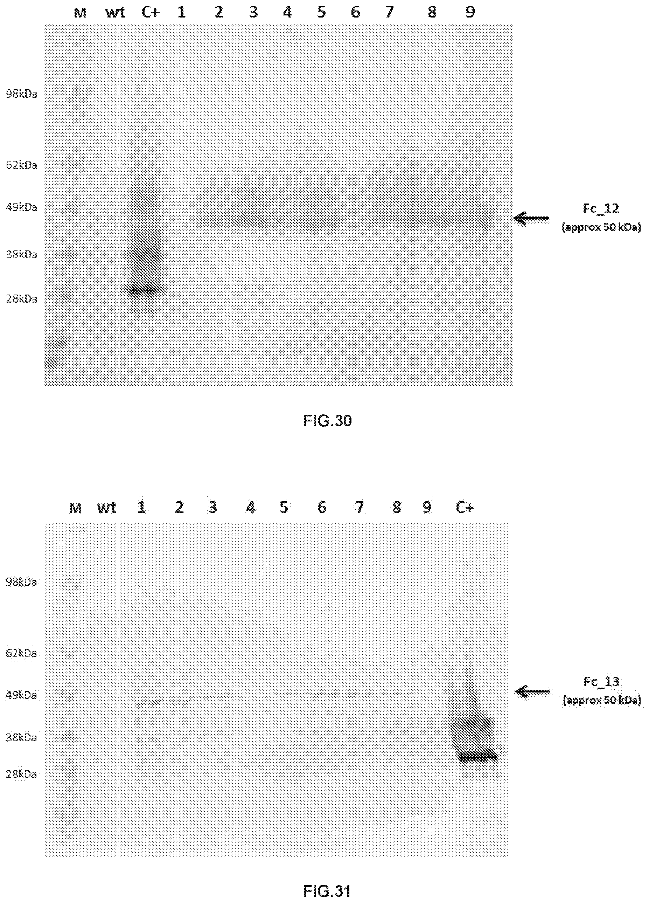

Western blot analysis showed GFP antibody detection in most transgenic plant lines for all fusion cassettes at the expected fusion protein size when compared to the SeeBlue® Plus2 Pre-Stained protein standard (Life Technologies, LC5925) as set out in Figs. 25 to 30. GFP antibody detects positive control, fluorescent protein (YPet), at approximately 28 kDa. Detection of the fusion cassettes using GFP antibody was specific, however, for some cassettes the sizes revealed to be slightly smaller than expected when compared to the protein standard marker. This could be due to the folding of the protein fusion complex within the endoplasmic reticulum. Nine (9) independent plant lines were analysed for each Fc, except for Fc_3 for which sixteen (16) plant lines were analysed.

### 2.2 Isolation and purification of SF-B peptide

Sequence analysis of purified His-tag protein extract in solution from construct Fc_9 (GFP-His-tag fusion cassette) and digested with trypsin, revealed the presence of three peptides: IQAMIPK, VVPLVAGGICQCLAER and YSVILLDTLLGR. Analysis of these peptides using UniProt and SwissPROT databases showed that all peptides scored 100% identity to mature human SP-B.

### REFERENCES

Almlen A, Walther FJ, Waring AJ, Robertson B, Johansson J, Curstedt T (2010) Synthetic surfactant based on analogues of SP-B and SP-C is superior to single-peptide surfactants in ventilated premature rabbits. Neonatology 98(1): 91-99
Blanco O, Lugones Y, Fernandez O, Faure R (2012) An update on clinical surfactant preparations and respiratory disease. Biotecnologia Aplicada 29: 53-59
Clark H, Clark LS (2005) The genetics of neonatal respiratory disease. Seminars in Fetal and Neonatal Medicine 10(3): 271-282
Cockshutt AM, Possmayer F (1992) Metabolism of surfactant lipids and proteins in the developing lung. In: Robertson B, van Golde LMG, Batenburg JJ (eds) Pulmonary surfactant: From Molecular Biology to Clinical Practice. Elsevier Science Publishers, Amsterdam, pp 339-378
Creuwels LAJM, Van Golde LMG, Haagsman HP (1997) The pulmonary surfactant system: Biochemical and clinical aspects. Lung 175: 1-39
Drugmand J-C, Schneider Y-J, Agathos SN (2012) Insect cells as factories for biomanufacturing. Biotechnology Advances 30(5): 1140-1157
Gleave AP (1992) A versatile binary vector system with a T-DNA organisational structure conducive to efficient integration of cloned DNA into the plant genome. Plant Molecular Biology 20: 1203-1207
Goerke J (1998) Pulmonary surfactant: functions and molecular composition. Biochem Biophys Acta 1408: 79-89
Guttentag S, Foster CD (2011) Update in surfactant therapy. NeoReviews 12: e625-e633
Sambrook J, Fritsch EF, Maniatis T (1989) Molecular Cloning: A laboratory manual. 2nd ed. Cold Spring Harbor Laboratory Press, Cold Spring Harbor.
Halliday HL (2008) Surfactants: past present and future. Journal of Perinatology 28: S47-S56
Wilson DF, Notter RH (2011) The future of exogenous surfactant therapy. Respiratory Care 56(9): 1369-1386
Ma CC-H, Ma S (2012) The role of surfactant in respiratory distress syndrome. The Open Respiratory Medicine Journal 6: 44-53
Pryhuber GS (1998) Regulation and function of pulmonary surfactant protein B. Molecular Genetics and Metabolism 64: 217-228
Jobe AH (1993) Pulmonary surfactant therapy. New England Journal of Medicine 328:861-868.
Mallory GB (2001) Surfactant proteins: Role in lung physiology and disease in early life. Paediatric Respiratory Reviews 2 (2): 151-158
Haataja R, Hallman M (2002) Surfactant proteins as genetic determinants of multifactorial pulmonary diseases. Annals of Medicine 34(5): 324-333
Whitsett JA, Weaver TE (2002) Hydrophobic surfactant proteins in lung function and disease. New England Journal of Medicine 347(26): 2141-2148
Griese M (1999) Pulmonary surfactant in health and human lung diseases: State of the art. European Respiratory Journal 13(6): 1455-1476
Weaver TE, Conkright JJ (2001) Functions of surfactant proteins B and C. Annual Review of Physiology 63: 555-578
Stevens TP, Sinkin RA (2007) Surfactant replacement therapy. Chest 131(5): 1577-1582
Finer NN (2004) Surfactant use for neonatal lung injury: Beyond respiratory distress syndrome. Paediatric Respiratory Reviews 5(SUPPL. A): S289-S297
Lusuardi M, Capelli A, Carli S, Tacconi MT, Salmona M, Donner CF (1992) Role of surfactant in chronic obstructive pulmonary disease: Therapeutic implications. Respiration 59(SUPPL. 1): 28-32
Kim JY, Kim Y-G, Lee GM (2012) CHO cells in biotechnology for production of recombinant proteins: Current state and further potential. Applied Microbiology and Biotechnology 93(3): 917-930
Mattanovich D, Branduardi P, Dato L, Gasser B, Sauer M, Porro D (2012) Recombinant protein production in yeasts. Methods in Molecular Biology 824: 329-358
Murashige T, Skoog F (1962) A revised medium for rapid growth and bio assays with tobacco tissue cultures. Plant Physiology 15:473-497
Huang C-J, Lin H, Yang X (2012) Industrial production of recombinant therapeutics in Escherichia coli and its recent advancements. Journal of Industrial Microbiology and Biotechnology 39(3): 383-399
Engle WA, Stark AR, Adamkin DH, Batton DG, Bell EF, Bhutani VK, Denson SE, Martin GI, Watterberg KL (2008) Surfactant-replacement therapy for respiratory distress in the preterm and term neonate. Pediatrics 121(2): 419-432
Seehase M, Collins JJP, Kuypers E, Jellema RK, Ophelders DRMG, Ospina OL, Perez-Gil J, Bianco F, Garzia R, Razzetti R, Kramer BW (2012) New surfactant with SP-B and SP-C analogs gives survival benefit after inactivation in preterm lambs. PLoS ONE 7(10): Article no.47631
Mingarro I, Lukovic D, Vilar M, Pérez-Gil J (2008) Synthetic pulmonary surfactant preparations: New developments and future trends. Current Medicinal Chemistry 15(4): 393-403
Mattanovich D, Ruker F, da Camara Machado A, Laimer M, Regner F, Steinkeliner H, Himmler G, Katinger H (1989) Efficient transformation of Agrobacterium spp. by eletroporation. Nucleic Acids Research 17(16):6747
Horsch R, Fry J, Hofmann N, Eichhlotz D, Rogers S, Fraylet R (1985) A simple and general method for transferring genes into plants. Science 227(4691): 1229-1231
Parra E, Moleiro LH, Lopez-Montero I, Cruz A, Monroy F, Perez-Gil J (2011) A combined action of pulmonary surfactant proteins SP-B and SP-C modulates permeability and dynamics of phospholipid membranes. Biochemical Journal 438(3): 555-564
Lukovic D, Plasencia I, Taberner FJ, Salgado J, Calvete JJ, Perez-Gil J, Mingarro I (2006) Production and characterisation of recombinant forms of human pulmonary surfactant protein C (SP-C): Structure and surface activity. Biochimica et Biophysica Acta - Biomembranes 1758(4): 509-518
Jordan BK, Donn SM (2013) Lucinactant for the prevention of respiratory distress syndrome in premature infants. Expert Review of Clinical Pharmacology 6(2): 115-121
Berlec A, Strukelj B (2013) Current state and recent advances in biopharmaceutical production in Escherichia coli, yeasts and mammalian cells. Journal of Industrial Microbiology and Biotechnology 40(3-4): 257-274
Yurdakök M (2004) Inherited disorders of neonatal lung diseases. Turkish Journal of Pediatrics 46(2): 105-114
Zuo YY, Veldhuizen RAW, Neumann AW, Petersen NO, Possmayer F (2008) Current perspectives in pulmonary surfactant - Inhibition, enhancement and evaluation. Biochimica et Biophysica Acta - Biomembranes 1778(10): 1947-1977
Hager, A.A., and DePaoli, T. (2001). Method for extracting and purifying pulmonary surfactant
Wang, W., Vignani, R., Scali, M., and Cresti, M. (2006). A universal and rapid protocol for protein extraction from recalcitrant plant tissues for proteomic analysis. Electrophoresis 27, 2782-2786.

## Claims

1. A method of producing a pulmonary surfactant protein-B (SP-B) mature peptide, in a plant cell, the method comprising the steps of:
(i) providing a nucleic acid sequence consisting of a polynucleotide sequence encoding a SP-B mature peptide, or analog thereof;
(ii) introducing the nucleic acid sequence into an expression vector adapted to express a polypeptide in a plant cell;
(iii) introducing the expression vector of step (ii) into a plant cell;
(iv) expressing the SP-B mature peptide, or analog thereof in the plant cell of step (iii); and
(v) harvesting the SP-B mature peptide, or analog thereof from the plant cell, wherein the encoded SP-B mature peptide sequence is set forth in SEQ ID NO: 3, or is a variant sequence at least 90% identical to SEQ ID NO: 3 and wherein the variant sequence is capable of lowering surface tension of a lipid bilayer membrane on interaction with phospholipids.

2. The method according to claim 1, wherein the polynucleotide sequence encoding the SP-B mature peptide is:
a) 100% identical to SEQ I.D. NO 4;
b) a variant sequence at least 80% identical to SEQ I.D. NO 4; or
c) a sequence which hybridises under stringent conditions to the reverse complement of of SEQ I.D. NO 4,
wherein the variant sequence or sequence which hybridises under stringent conditions to the reverse complement encodes a polypeptide capable of lowering surface tension of a lipid bilayer membrane on interaction with phospholipids.

3. The method according to either claim 2 or 3, wherein the number of hydrophobic amino acid residues encoded by the variant sequence or sequence which hybridises under stringent conditions to the reverse complement of SEQ I.D. NO 4, or the number of hydrophobic amino acid residues in the variant sequence of of SEQ I.D. NO 3 is the same as, or greater than, the number of hydrophobic amino acid residues encoded by SEQ I.D. NO 4 or of SEQ I.D. NO 3.

4. The method according to any one of claims 1 to 3 wherein said SP-B mature polypeptide or analog thereof is fused to a heterologous polypeptide to improve expression levels and purification efficiencies of the SP-B mature peptide or analog thereof in a plant cell.

5. The method according to of claim 4, wherein the nucleic acid sequence encodes any one or more of the following heterologous polypeptide elements:
(i) a polynucleotide sequence encoding a protease cleavage site, including an enterokinase, chymosin or Tobacco Etch Virus (TEV) protease cleavage site;
(ii) a polynucleotide sequence encoding a tag including a polyhistidine, leptin, late embryogenesis abundant protein (LEA), lectin, maltose binding protein (MBP), or glutathione S-transferase (GST) tag;
(iii) a polynucleotide sequence encoding a marker protein for detection, including YPet or GFP;
(iv) a plant promoter including chrysanthemum RbcS1, 35S, including CaMV35S, and a corresponding terminator sequence;
(v) a polynucleotide sequence encoding a signal peptide including the signal peptide region of equistatin or of *Nicotiana tabacum* thionin (NtSP); and
(vi) a polynucleotide sequence encoding an endoplasmic reticulum (ER) trafficking peptide including SEKDEL or KDEL; an oil body- trafficking peptide including oleosin; a protein storage vacuole-trafficking peptide including Vacuolar Sorting Determinant (VSD); or a plastid, including a chloroplast, chromoplast or leucoplast-trafficking peptide including a peptide capable of interacting with the thylakoid membrane of a plastid, or a polynucleotide sequence comprising a psbA regulatory 5'-UTR and 3' UTR region for targeting an encoded transcript to a plastid.

6. The method according to claim 5, wherein the polynucleotide sequence encoding the SP-B mature peptide, or analog thereof is operably linked in a fusion cassette together with one or more elements including: MBP; YPet; chymosin; lectin; TEV; polyhistidine; leptin; or LEA.

7. The method according to claim 6, wherein the fusion cassette consists of any one of the following combinations in the order set out:
(i) MBP - YPet - chymosin - SP-B;
(ii) lectin - YPet - TEV - SP-B - TEV;
(iii) YPet - TEV - SP-B - TEV - polyhistidine;
(iv) YPet - chymosin - SP-B - TEV - leptin;
(v) LEA - chymosin - SP-B - TEV - YPet; and
(vi) YPet - chymosin - SP-B - TEV - LEA.

8. The method according any one of claims 1 to 7, wherein the expression vector of step (ii) further comprises a polynucleotide sequence encoding a suppressor protein adapted to inhibit post-transcriptional gene silencing in a plant cell, or where step (iii) further includes introducing into the plant cell a second plant vector comprising a polynucleotide sequence encoding a suppressor protein adapted to inhibit post-transcriptional gene silencing in the plant cell, the suppressor protein selected from the NSs protein of the tomato spotted wilt virus or the p19 of tomato bushy stunt virus.

9. The method according to claim 1, wherein the plant expression vector of step (ii) is an *Agrobacterium tumefaciens* vector.

10. The method according to claim 9, wherein step (iii) of the method comprises stable transformation of a plant cell by the introduction of the expression vector of step (ii) into the genetic material of the plant cell by means of *Agrobacterium* transformation or agroinfiltration.

11. The method according to any one of claims 1 to 10, wherein the plant cell is a plurality of plant cells in suspension culture, plant cells in tissue culture, plant cells in a leaf of a plant or a transgenic plant or any part thereof.

12. A polypeptide fusion cassette comprising a SP-B mature peptide, or analog thereof produced by the method according to any one of claims 6 to 11 wherein the SP-B mature peptide sequence is set forth in SEQ ID NO: 3, or is a variant sequence at least 90% identical to SEQ ID NO: 3 and wherein the variant sequence is capable of lowering surface tension of a lipid bilayer membrane on interaction with phospholipids.

13. The polypeptide fusion cassette according to claim 12 wherein said cassette comprises a SP-B mature peptide or analog thereof fused to a heterologous polypeptide to improve expression levels and purification efficiencies of the SP-B mature peptide or analog thereof, in a plant cell.

14. A plant-based expression vector comprising a nucleic acid sequence as described in the method according to any one of claims 1 to 10.

15. A plant cell comprising a SP-B mature peptide, or analog thereof produced according to the method according to any one of claims 1 to 11, wherein the SP-B mature peptide sequence is set forth in SEQ ID NO: 3, or is a variant sequence at least 90% identical to SEQ ID NO: 3 and wherein the variant sequence is capable of lowering surface tension of a lipid bilayer membrane on interaction with phospholipids.

16. The plant cell according to claim 15 wherein the SP-B mature sequence or analog thereof is fused to a heterologous polypeptide to improve expression levels and purification efficiencies of the SP-B mature peptide or analogue thereof, in a plant cell.

## Patentansprüche

1. Verfahren zur Herstellung eines reifen oberflächenaktiven Lungenprotein-B-Peptids (SP-B-Peptids), umfassend die folgenden Schritte:
(i) die Bereitstellung eines Nukleinsäurestrangs bestehend aus einem ein reifes SP-B-Peptid codierenden Polynukleotidstrang oder einem Analogon desselben;
(ii) die Einführung des Nukleinsäurestrangs in einen Expressionsvektors, der zur Expression eines Polypeptids in einer Pflanzenzelle ausgelegt ist;
(iii) die Einführung des Expressionsvektors aus Schritt (ii) in eine Pflanzenzelle;
(iv) die Expression des reifen SP-B-Peptid oder eines Analogons desselben in die Pflanzenzelle aus Schritt (iii); und
(v) das Ernten des reifen SP-B-Peptid oder eines Analogons desselben in der Pflanzenzelle von Schritt (iii), wobei der codierte reife SP-B-Peptidstrang in SEQ ID Nr. 3 dargelegt wird oder ein mindestens 90% mit SEQ ID Nr. 3 identischer Variantenstrang ist, und wobei der Variantenstrang bei Wechselwirkung mit Phospholipiden die Oberflächenspannung einer Lipidbilayermembran verringern kann.

2. Verfahren nach Anspruch 1, wobei der das reife SP-B-Peptid codierende Polynukleotidstrang wie folgt beschaffen ist:
a) 100% identisch mit SEQ ID Nr. 4;
b) ein mindestens 80% mit SEQ ID Nr. 4 identischer Variantenstrang; oder
c) ein Strang, der sich unter strikten Bedingungen zum Umkehrkomplement von SEQ ID Nr. 4 hybridisiert,
wobei der Variantenstrang oder der Strang, der sich unter strikten Bedingungen zum Umkehrkomplement hybridisiert, ein Polypeptid codiert, das bei Wechselwirkung mit Phospholipiden die Oberflächenspannung einer Lipidbilayermembran verringern kann.

3. Verfahren nach Anspruch 2 oder 3, wobei die Anzahl der vom Variantenstrang oder von dem Strang, der sich unter strikten Bedingungen zum Umkehrkomplement von SEQ ID Nr. 4 hybridisiert, codierten hydrophoben Aminsäurereste bzw. die Anzahl der hydrophoben Aminsäurereste im Variantenstrang von SEQ ID Nr. 3 gleich wie oder größer ist als die Anzahl der von SEQ ID Nr. 4 oder SEQ ID Nr. 3 codierten hydrophoben Aminsäurereste.

4. Verfahren nach einem der Ansprüche 1 bis 3, wobei das besagte reife SP-B-Polypeptid oder das Analogon desselben zur Verbesserung der Expressionswerte und der Reinigungsleistung des reifen SP-B-Peptids oder des Analogons desselben in einer Pflanzenzelle mit einem hetorologen Polypeptid verschmolzen wird.

5. Verfahren nach Anspruch 4, wobei der Nukleinsäurestrang eines oder mehr der folgenden hetorologen Polypeptidelemente codiert:
(i) einen Polynukleotidstrang, der eine Proteasespaltstelle, einschließlich einer Enterokinase-, Chymosin- oder Tabakätzvirus-Proteasespaltstelle (TEV), codiert;
(ii) einen Polynukleotidstrang, der eine Marke, einschließlich einer Polyhistidin-, Leptin-, Spätembryogenese-Abundantprotein- (LEA), Lectin-, Maltosebindungsprotein- (MBP) oder Glutathion-S-Transferase-(GST) Marke, codiert;
(iii) einen Polynukleotidstrang, der ein Markierungsprotein für Detektion, einschließlich von YPet oder GFP, codiert;
(iv) einen Pflanzenaktivator, einschließlich von Chrysanthemen-RbcS1, 35S, einschließlich von CaMV35S, und eines entsprechenden Abbrechstrangs;
(v) einen Polynukleotidstrang, der ein Signalpeptid, einschließlich des Signalpeptidbereichs von Äquistatin oder *Nicotiana tabacum*-Thionin (NtSP), codiert; und
(vi) einen Polynukleotidstrang, der ein endoplasmatisches Retikulum- (ER) Transportpeptid einschließlich von SEKDEL oder KDEL; ein Ölkörper-Transportpeptid einschließlich von Oleosin; ein Proteinspeichervakuole-Transportpeptid einschließlich von Vakuolär-Sortierdeterminante (VSD); oder ein Plastid einschließlich eines Chloroplast-, Chromoplast- oder Leukoplast-Transportpeptids einschließlich eines Peptids, das zur Wechselwirkung mit der Thylalkoidmembran eines Plastids befähigt ist, oder eines Polynukleotidstrangs mit einem psbA-Regel-5'UTR- und 3'UTR-Bereich zur Anpeilung eines codierten Transkripts auf ein Plastid codiert.

6. Verfahren nach Anspruch 5, wobei der das reife SP-B-Peptid oder ein Analogon desselben codierende Polynukleotidstrang in einer Fusionskassette in Wirkvernindung mit einem oder mehr der Elemente MBP; YPet; Chymosin; Lectin; TEV; Polyhistidin; Leptin; oder LEA gebracht wird.

7. Verfahren nach Anspruch 6, wobei die Fusionskassette in der angegebenen Reihenfolge aus einer beliebigen der folgenden Kombinationen besteht:
(i) MBP - YPet - Chrymosin - SP-B;
(ii) Lectin - YPet - TEV - SP-B - TEV;
(iii) YPet - TEV - SP-B - TEV - Polyhistidin;
(iv) YPet - Chrymosin - SP-B - TEV - Leptin;
(v) LEA - Chrymosin - SP-B - TEV - YPet; und
(vi) YPet - Chrymosin - SP-B - TEV - LEA.

8. Verfahren nach einem der Ansprüche 1 bis 7, wobei der Expressionsvektor von Schritt (ii) weiter einen Polynukleotidstrang umfasst, der ein zur Hemmung der posttranskriptionalen Gendämmung in einer Pflanzenzelle ausgelegtes Suppressorprotein codiert, oder wobei Schritt (iii) weiter die Einführung eines zweiten Pflanzenvektors in die Pflanzentelle umfasst, der einen Polynukleotidstrang umfasst, der ein zur Hemmung der posttranskriptionalen Gendämmung in einer Pflanzenzelle ausgelegtes Suppressorprotein codiert, wobei das Suppressorprotein aus dem NSs-Protein der mit dem Tomatenbronzefleckenvirus oder dem p19 Tomatenzwergbuschvirus befallenen Tomate ausgewählt wird.

9. Verfahren nach Anspruch 1, wobei der Pflanzenexpressionsvektor von Schritt (ii) ein *Agrobacterium tumefaciens-Vektor* ist.

10. Verfahren nach Schritt 9, wobei Schritt (iii) des Verfahrens die stabile Transformatiom einer Pflanzenelle durch die Einführung des Expressionsvektors von Schritt (ii) in das genetische Material der Pflanze mittels *Agrobacterium-*Transformation oder Agroinfiltration umfasst.

11. Verfahren nach einem der Ansprüche 1 bis 10, wobei die Pflanzenzelle eine Vielzahl von Pflanzenzellen in Suspensionskultur, Pflanzenzellen in Gewebekultur, Pflanzenzellen in einem Blatt einer Pflanze oder einer transgenen Pflanze oder eines Teils derselben ist.

12. Polypeptidfusionskassette umfassend ein mittels des Verfahrens nach einem der Ansprüche 6 bis 11 hergestelltes reifes SP-B-Peptid oder ein Analogons desselben, wobei der reife SP-B-Peptidstrang in SEQ ID Nr. 3 dargelegt wird oder ein mindestens 90% mit SEQ ID Nr. 3 identischer Variantenstrang ist, und wobei der Variantenstrang bei Wechselwirkung mit Phospholipiden die Oberflächenspannung einer Lipidbilayermembran verringern kann.

13. Polypeptidfusionskassette nach Anspruch 12, wobei die besagte Kassette ein des reifes SP-B-Peptid oder ein Analogon desselben umfasst, das zur Verbesserung der Expressionswerte und der Reinigungsleistung des reifen SP-B-Peptids oder des Analogons desselben zu einem heterologen Polypeptid verschmolzen wird.

14. Auf Pflanzen basierender Expressionsvektor, umfassend einen im Verfahren nach einem der Ansprüche 1 bis 10 beschriebenen Nukleinsäurestrang.

15. Pflanzenzelle, umfassend ein gemäß dem Verfahren nach einem der Ansprüche 1 bis 11 hergestelltes reifes SP-B-Peptid oder ein Analogon desselben, wobei der reife SP-B-Peptidstrang in SEQ ID Nr. 3 dargelegt wird oder ein mindestens 90% mit SEQ ID Nr. 3 identischer Variantenstrang ist, und wobei der Variantenstrang bei Wechselwirkung mit Phospholipiden die Oberflächenspannung einer Lipidbilayermembran verringern kann.

16. Pflanzenzelle nach Anspruch 15, wobei der reife SP-B-Peptidstrang oder ein Analogon desselben zur Verbesserung der Expressionswerte und der Reinigungsleistung des reifen SP-B-Peptids oder des Analogons desselben zu einem heterologen Polypeptid verschmolzen wird.

## Revendications

1. Procédé de production d'un peptide mûr de protéine B du surfactant pulmonaire (SP-B) dans une cellule végétale, le procédé comprenant les étapes consistant à :
(i) fournir une séquence d'acide nucléique constituée d'une séquence polynucléotidique codant pour un peptide mûr de SP-B ou analogue de celui-ci ;
(ii) introduire la séquence d'acide nucléique dans un vecteur d'expression adapté à exprimer un polypeptide dans une cellule végétale ;
(iii) introduire le vecteur d'expression de l'étape (ii) dans une cellule végétale ;
(iv) exprimer le peptide mûr de SP-B ou analogue de celui-ci dans la cellule végétale de l'étape (iii) ; et
(v) récolter le peptide mûr de SP-B ou analogue de celui-ci de la cellule végétale, dans lequel la séquence de peptide mûr de SP-B codée est établie dans SEQ ID N° : 3 ou est une variante de séquence identique à au moins 90 % à SEQ ID N° : 3, et dans lequel la variante de séquence est capable d'abaisser la tension superficielle d'une membrane lipidique bicouches lors de l'interaction avec des phospholipides.

2. Procédé selon la revendication 1, dans lequel la séquence polynucléotidique codant pour le peptide mûr de SP-B est :
a) identique à 100 % à SEQ ID N° 4 ;
b) une variante de séquence identique à au moins 80 % à SEQ ID N° 4 ; ou
c) une séquence qui s'hybride dans des conditions strictes au complément inverse de SEQ ID N° 4,
dans lequel la variante de séquence ou séquence qui s'hybride dans des conditions strictes au complément inverse code pour un polypeptide capable d'abaisser la tension superficielle d'une membrane lipidique bicouches lors de l'interaction avec des phospholipides.

3. Procédé selon la revendication 2 ou 3, dans lequel le nombre de résidus d'acide aminé hydrophobes codés par la variante de séquence ou séquence qui s'hybride dans des conditions strictes au complément inverse de SEQ ID N° 4 ou le nombre de résidus d'acide aminé hydrophobes dans la variante de séquence de SEQ ID N° 3 est le même que, ou supérieur à, le nombre de résidus d'acide aminé hydrophobes codés par SEQ ID N° 4 ou de SEQ ID N° 3.

4. Procédé selon l'une quelconque des revendications 1 à 3, dans lequel ledit polypeptide mûr de SP-B ou analogue de celui-ci est fusionné à un polypeptide hétérologue pour améliorer les niveaux d'expression et efficacités de purification du peptide mûr de SP-B ou analogue de celui-ci dans une cellule végétale.

5. Procédé selon la revendication 4, dans lequel la séquence d'acide nucléique code pour l'un quelconque ou plusieurs des éléments de polypeptide hétérologues suivants :
(i) une séquence polynucléotidique codant pour un site de clivage de protéase, y compris un site de clivage de protéase d'entérokinase, de chymosine ou du virus de la gravure du tabac (TEV) ;
(ii) une séquence polynucléotidique codant pour une étiquette incluant une étiquette polyhistidine, leptine, protéine abondante de l'embryogenèse tardive (LEA), lectine, protéine de liaison du maltose (MBP) ou glutathion S-transférase (GST) ;
(iii) une séquence polynucléotidique codant pour une protéine de marqueur pour la détection, y compris YPet ou GFP ;
(iv) un promoteur végétal incluant RbcS1 de chrysanthème, 35S, y compris CaMV35S et une séquence terminatrice correspondante ;
(v) une séquence polynucléotidique codant pour un peptide de signal incluant la région de peptide de signal d'équistatine ou de thionine de Nicotiana tabacum (NtSP) ;
et
(vi) une séquence polynucléotidique codant pour un peptide d'acheminement de réticulum endoplasmique (ER) incluant SEKDEL ou KDEL ; un peptide d'acheminement de corps huileux incluant l'oléosine ; un peptide d'acheminement de vacuole de stockage protéique incluant le déterminant de tri vacuolaire (VSD) ; ou un plastide, y compris un chloroplaste, chromoplaste ou peptide d'acheminement de leucoplaste incluant un peptide capable d'interagir avec la membrane thylacoïde d'un plastide, ou une séquence polynucléotidique comprenant une région 5'-UTR et 3'-UTR régulatrice de psbA pour cibler un produit de la transcription codé vers un plastide.

6. Procédé selon la revendication 5, dans lequel la séquence polynucléotidique codant pour le peptide mûr de SP-B ou analogue de celui-ci est liée de manière opérationnelle dans une cassette de fusion à un ou plusieurs éléments incluant : MBP ; YPet ; chymosine ; lectine, TEV ; polyhistidine ; leptine ; ou LEA.

7. Procédé selon la revendication 6, dans lequel la cassette de fusion est constituée de l'une quelconque des combinaisons suivantes dans l'ordre établi :
(i) MBP - YPet - chymosine - SP-B ;
(ii) lectine - YPet -TEV - SP-B - TEV ;
(iii) YPet - TEV - SP-B - TEV - polyhistidine ;
(iv) YPet - chymosine - SP-B - TEV - leptine ;
(v) LEA - chymosine - SP-B - TEV - YPet ; et
(vi) YPet - chymosine - SP-B - TEV - LEA.

8. Procédé selon l'une quelconque des revendications 1 à 7, dans lequel le vecteur d'expression de l'étape (ii) comprend en outre une séquence polynucléotidique codant pour une protéine suppressive adaptée à inhiber le silençage génique post-transcriptionnel dans une cellule végétale, ou où l'étape (iii) inclut en outre introduire dans la cellule végétale un second vecteur végétal comprenant une séquence polynucléotidique codant pour une protéine suppressive adaptée à inhiber le silençage génique post-transcriptionnel dans la cellule végétale, la protéine suppressive étant sélectionnée parmi la protéine NSs du virus des taches bronzées de la tomate ou la p19 du virus du nanisme rabougri de la tomate.

9. Procédé selon la revendication 1, dans lequel le vecteur d'expression végétal de l'étape (ii) est un vecteur d'Agrobacterium tumefaciens.

10. Procédé selon la revendication 9, dans lequel l'étape (iii) du procédé comprend la transformation stable d'une cellule végétale par l'introduction du vecteur d'expression de l'étape (ii) dans le matériau génétique de la cellule végétale au moyen d'une transformation ou agroinfiltration d'Agrobacterium.

11. Procédé selon l'une quelconque des revendications 1 à 10, dans lequel la cellule végétale est une pluralité de cellules végétales en culture de suspension, de cellules végétales en culture de tissu, de cellules végétale dans une feuille d'une plante ou d'une plante transgénique ou de toute partie de celle-ci.

12. Cassette de fusion de polypeptide comprenant un peptide mûr de SP-B ou analogue de celui-ci produit par le procédé selon l'une quelconque des revendications 6 à 11, dans laquelle la séquence de peptide mûr de SP-B est établie dans SEQ ID N° : 3 ou est une variante de séquence identique à au moins 90 % à SEQ ID N° : 3, et dans laquelle la variante de séquence est capable d'abaisser la tension superficielle d'une membrane lipidique bicouches lors de l'interaction avec des phospholipides.

13. Cassette de fusion de polypeptide selon la revendication 12, dans laquelle ladite cassette comprend un peptide mûr de SP-B ou analogue de celui-ci fusionné à un polypeptide hétérologue pour améliorer les niveaux d'expression et efficacités de purification du peptide mûr de SP-B ou analogue de celui-ci dans une cellule végétale.

14. Vecteur d'expression à base végétale comprenant une séquence d'acide nucléique telle que décrite dans le procédé selon l'une quelconque des revendications 1 à 10.

15. Cellule végétale comprenant un peptide mûr de SP-B ou analogue de celui-ci produit selon le procédé selon l'une quelconque des revendications 1 à 11, dans laquelle la séquence de peptide mûr de SP-B est établie dans SEQ ID N° : 3 ou est une variante de séquence identique à au moins 90 % à SEQ ID N° : 3, et dans laquelle la variante de séquence est capable d'abaisser la tension superficielle d'une membrane lipidique bicouches lors de l'interaction avec des phospholipides.

16. Cellule végétale selon la revendication 15, dans laquelle la séquence mûre de SP-B ou analogue de celle-ci est fusionnée à un polypeptide hétérologue pour améliorer les niveaux d'expression et efficacités de purification du peptide mûr de SP-B ou analogue de celui-ci dans une cellule végétale.


### SEQUENCE LISTING

<110> Azargen Biotechnologies (Pty) Ltd
<120> PRODUCTION OF HUMAN PULMONARY SURFACTANT PROTEIN B IN PLANTS
<130> P1148PC00
<150> 2013/04666
   <151> 2013-06-24
<160> 30
<170> PatentIn version 3.5
<210> 1
   <211> 381
   <212> PRT
   <213> Homo sapiens
<400> 1
<210> 2
   <211> 1146
   <212> DNA
   <213> Homo sapiens
<400> 2
   atggctgagt cacacctgct gcagtggctg ctgctgctgc tgcccacgct ctgtggccca      60
   ggcactgctg cctggaccac ctcatccttg gcctgtgccc agggccctga gttctggtgc     120
   caaagcctgg agcaagcatt gcagtgcaga gccctagggc attgcctaca ggaagtctgg     180
   ggacatgtgg gagccgatga cctatgccaa gagtgtgagg acatcgtcca catccttaac     240
   aagatggcca aggaggccat tttccaggac acgatgagga agttcctgga gcaggagtgc     300
   aacgtcctcc ccttgaagct gctcatgccc cagtgcaacc aagtgcttga cgactacttc     360
   cccctggtca tcgactactt ccagaaccag actgactcaa acggcatctg tatgcacctg     420
   ggcctgtgca aatcccggca gccagagcca gagcaggagc cagggatgtc agaccccctg    480
   cccaaacctc tgcgggaccc tctgccagac cctctgctgg acaagctcgt cctccctgtg     540
   ctgcccgggg ccctccaggc gaggcctggg cctcacacac aggatctctc cgagcagcaa     600
   ttccccattc ctctccccta ttgctggctc tgcagggctc tgatcaagcg gatccaagcc     660
   atgattccca agggtgcgct agctgtggca gtggcccagg tgtgccgcgt ggtacctctg     720
   gtggcgggcg gcatctgcca gtgcctggct gagcgctact ccgtcatcct gctcgacacg     780
   ctgctgggcc gcatgctgcc ccagctggtc tgccgcctcg tcctccggtg ctccatggat     840
   gacagcgctg gcccaaggtc gccgacagga gaatggctgc cgcgagactc tgagtgccac     900
   ctctgcatgt ccgtgaccac ccaggccggg aacagcagcg agcaggccat accacaggca     960
   atgctccagg cctgtgttgg ctcctggctg gacagggaaa agtgcaagca atttgtggag    1020
   cagcacacgc cccagctgct gaccctggtg cccaggggct gggatgccca caccacctgc    1080
   caggccctcg ggtgtgtggg gaccatgtcc agccctctcc agtgtatcca cagccccgac    1140
   ctttga                                                               1146
<210> 3
   <211> 79
   <212> PRT
   <213> Homo sapiens
<400> 3
   Phe Pro Ile Pro Leu Pro Tyr Cys Trp Leu Cys Arg Ala Leu Ile Lys
   1               5                   10                  15
   Arg Ile Gln Ala Met Ile Pro Lys Gly Ala Leu Ala Val Ala Val Ala
               20                  25                  30
   Gln Val Cys Arg Val Val Pro Leu Val Ala Gly Gly Ile Cys Gln Cys
           35                  40                  45
   Leu Ala Glu Arg Tyr Ser Val Ile Leu Leu Asp Thr Leu Leu Gly Arg
       50                  55                  60
   Met Leu Pro Gln Leu Val Cys Arg Leu Val Leu Arg Cys Ser Met
               65                  70                  75
<210> 4
   <211> 237
   <212> DNA
   <213> Homo sapiens
<400> 4
   ttccccattc ctctccccta ttgctggctc tgcagggctc tgatcaagcg gatccaagcc        60
   atgattccca agggtgcgct agctgtggca gtggcccagg tgtgccgcgt ggtacctctg       120
   gtggcgggcg gcatctgcca gtgcctggct gagcgctact ccgtcatcct gctcgacacg       180
   ctgctgggcc gcatgctgcc ccagctggtc tgccgcctcg tcctccggtg ctccatg          237
<210> 5
   <211> 2115
   <212> DNA
   <213> Artificial Sequence
<220>
   <223> cDNA sequence encoding the fusion cassette consisting of MBP - YPet - chymosin - SP-B
<400> 5
   gctaagattg aagaaggaaa actcgtgata tggattaatg gcgacaaagg ctataacggt 60
   ttggctgagg ttgggaagaa attcgaaaaa gataccggta taaaggttac tgtggagcac 120
   ccagacaaac tggaagagaa atttccacaa gttgccgcca ctggcgacgg tcctgatatc 180
   attttctggg ctcacgatag gtttggcgga tatgcacagt ctggcttgtt ggcagaaatt 240
   acccctgata aagccttcca agataagctg taccctttta catgggatgc agtgagatac 300
   aatggtaaac taatagctta tcctattgct gtggaggctt tgtcactcat ctacaataag 360
   gaccttttgc ctaatccacc taagacttgg gaagagatac ctgctctaga caaagagttg 420
   aaagcaaagg gaaagtccgc tcttatgttt aaccttcaag aaccatactt tacttggcca 480
   ctgattgccg ctgatggtgg gtacgctttt aagtatgaaa atgggaagta cgatatcaaa 540
   gatgtgggag tagataatgc tggcgctaaa gctggtctta cattcttggt cgatttgata 600
   aagaataagc atatgaatgc agacaccgac tatagcatag ctgaggctgc cttcaataag 660
   ggtgagactg ccatgactat taacggaccc tgggcttggt ctaacattga cactagtaaa 720
   gttaactatg gagtaaccgt actacctact tttaagggtc agccttctaa gccttttgtg 780
   ggggttttat ccgctggtat taacgctgca tcacctaata aggagttagc aaaagagttt 840
   cttgagaatt acttactcac tgatgagggt ctggaagcag ttaacaaaga taaacctttg 900
   ggcgctgtgg cactcaaatc atatgaggaa gaactagcta aagatccaag gattgctgct 960
   acaatggaga atgcccagaa aggagagatt atgccaaaca ttccccaaat gagcgccttt 1020
   tggtatgctg ttagaacagc cgtcatcaat gcagctagcg gccgtcaaac agttgacgaa 1080
   gctctcaagg acgctcagac aagaatcacc aaaatgtcta aaggagaaga attgtttact 1140
   ggtgtagtgc ctatccttgt tgaattggat ggtgatgtta atggccacaa attctcagta 1200
   agtggggaag gtgaaggcga cgctacatat ggaaagctca ctcttaagct gctatgtact 1260
   actggaaagt tgcccgtccc ttggccaaca ttggttacaa cacttggata tggggttcag 1320
   tgttttgcaa gatatccaga ccacatgaag caacatgact tcttcaaatc tgctatgcca 1380
   gagggatatg tccaagaaag gacaatattc ttcaaggatg atggaaatta caagacaaga 1440
   gctgaggtga agtttgaagg cgatacactg gttaacagaa tcgaactaaa gggcattgat 1500
   ttcaaggaag atggaaatat cttagggcat aagctggagt ataactataa ctctcataat 1560
   gtttacataa ccgcagacaa gcagaaaaac gggattaagg caaatttcaa gattaggcat 1620
   aacatcgagg atggaggtgt tcagctggca gaccattatc agcaaaatac accaataggt 1680
   gatggcccag tcttattgcc tgataaccat tatctctcct atcagagtgc tctctttaag 1740
   gaccctaacg aaaagagaga tcatatggtc ttacttgagt tcttgactgc agccggaatt 1800
   accgaaggga tgaacgaact ttacaagtgt acagaggact ttctacaaaa acaacaatat 1860
   ggaataagct caaaattctt ccctatccca ctgccttact gctggctttg tcgagccctt 1920
   atcaagagga ttcaagcaat gatcccaaaa ggtgccttag cagtcgccgt ggcccaagtt 1980
   tgcagagtag tgcctcttgt tgctggcgga atttgccaat gtcttgcaga gcgttacagc 2040
   gttattctgt tggatactct gttgggcaga atgctacctc aattggtgtg ccgtctggtg 2100
   ttacgttgta gcatg 2115
<210> 6
   <211> 705
   <212> PRT
   <213> Artificial Sequence
<220>
   <223> polypeptide sequence of the the fusion cassette consisting of MBP - YPet - chymosin - SP-B
<400> 6
   Ala Lys Ile Glu Glu Gly Lys Leu Val Ile Trp Ile Asn Gly Asp Lys
   1               5                   10                  15
   Gly Tyr Asn Gly Leu Ala Glu Val Gly Lys Lys Phe Glu Lys Asp Thr
               20                  25                  30
   Gly Ile Lys Val Thr Val Glu His Pro Asp Lys Leu Glu Glu Lys Phe
           35                  40                  45
   Pro Gln Val Ala Ala Thr Gly Asp Gly Pro Asp Ile Ile Phe Trp Ala
       50                  55                  60
   His Asp Arg Phe Gly Gly Tyr Ala Gln Ser Gly Leu Leu Ala Glu Ile
   65                  70                  75                  80
   Thr Pro Asp Lys Ala Phe Gln Asp Lys Leu Tyr Pro Phe Thr Trp Asp
                   85                  90                  95
   Ala Val Arg Tyr Asn Gly Lys Leu Ile Ala Tyr Pro Ile Ala Val Glu
               100                 105                 110
   Ala Leu Ser Leu Ile Tyr Asn Lys Asp Leu Leu Pro Asn Pro Pro Lys
           115                 120                 125
   Thr Trp Glu Glu Ile Pro Ala Leu Asp Lys Glu Leu Lys Ala Lys Gly
       130                 135                 140
   Lys Ser Ala Leu Met Phe Asn Leu Gln Glu Pro Tyr Phe Thr Trp Pro
   145                 150                 155                 160
   Leu Ile Ala Ala Asp Gly Gly Tyr Ala Phe Lys Tyr Glu Asn Gly Lys
                   165                 170                 175
   Tyr Asp Ile Lys Asp Val Gly Val Asp Asn Ala Gly Ala Lys Ala Gly
               180                 185                 190
   Leu Thr Phe Leu Val Asp Leu Ile Lys Asn Lys His Met Asn Ala Asp
           195                 200                 205
   Thr Asp Tyr Ser Ile Ala Glu Ala Ala Phe Asn Lys Gly Glu Thr Ala
       210                 215                 220
   Met Thr Ile Asn Gly Pro Trp Ala Trp Ser Asn Ile Asp Thr Ser Lys
   225                 230                 235                 240
   Val Asn Tyr Gly Val Thr Val Leu Pro Thr Phe Lys Gly Gln Pro Ser
                   245                 250                 255
   Lys Pro Phe Val Gly Val Leu Ser Ala Gly Ile Asn Ala Ala Ser Pro
               260                 265                 270
   Asn Lys Glu Leu Ala Lys Glu Phe Leu Glu Asn Tyr Leu Leu Thr Asp
           275                 280                 285
   Glu Gly Leu Glu Ala Val Asn Lys Asp Lys Pro Leu Gly Ala Val Ala
       290                 295                 300
   Leu Lys Ser Tyr Glu Glu Glu Leu Ala Lys Asp Pro Arg Ile Ala Ala
   305                 310                 315                 320
   Thr Met Glu Asn Ala Gln Lys Gly Glu Ile Met Pro Asn Ile Pro Gln
                   325                 330                 335
   Met Ser Ala Phe Trp Tyr Ala Val Arg Thr Ala Val Ile Asn Ala Ala
               340                 345                 350
   Ser Gly Arg Gln Thr Val Asp Glu Ala Leu Lys Asp Ala Gln Thr Arg
           355                 360                 365
   Ile Thr Lys Met Ser Lys Gly Glu Glu Leu Phe Thr Gly Val Val Pro
       370                 375                 380
   Ile Leu Val Glu Leu Asp Gly Asp Val Asn Gly His Lys Phe Ser Val
   385                 390                 395                 400
   Ser Gly Glu Gly Glu Gly Asp Ala Thr Tyr Gly Lys Leu Thr Leu Lys
                   405                 410                 415
   Leu Leu Cys Thr Thr Gly Lys Leu Pro Val Pro Trp Pro Thr Leu Val
               420                 425                 430
   Thr Thr Leu Gly Tyr Gly Val Gln Cys Phe Ala Arg Tyr Pro Asp His
           435                 440                 445
   Met Lys Gln His Asp Phe Phe Lys Ser Ala Met Pro Glu Gly Tyr Val
       450                 455                 460
   Gln Glu Arg Thr Ile Phe Phe Lys Asp Asp Gly Asn Tyr Lys Thr Arg
   465                 470                 475                 480
   Ala Glu Val Lys Phe Glu Gly Asp Thr Leu Val Asn Arg Ile Glu Leu
                   485                 490                 495
   Lys Gly Ile Asp Phe Lys Glu Asp Gly Asn Ile Leu Gly His Lys Leu
               500                 505                 510
   Glu Tyr Asn Tyr Asn Ser His Asn Val Tyr Ile Thr Ala Asp Lys Gln
           515                 520                 525
   Lys Asn Gly Ile Lys Ala Asn Phe Lys Ile Arg His Asn Ile Glu Asp
       530                 535                 540
   Gly Gly Val Gln Leu Ala Asp His Tyr Gln Gln Asn Thr Pro Ile Gly
   545                 550                 555                 560
   Asp Gly Pro Val Leu Leu Pro Asp Asn His Tyr Leu Ser Tyr Gln Ser
                   565                 570                 575
   Ala Leu Phe Lys Asp Pro Asn Glu Lys Arg Asp His Met Val Leu Leu
               580                 585                 590
   Glu Phe Leu Thr Ala Ala Gly Ile Thr Glu Gly Met Asn Glu Leu Tyr
           595                 600                 605
   Lys Cys Thr Glu Asp Phe Leu Gln Lys Gln Gln Tyr Gly Ile Ser Ser
       610                 615                 620
   Lys Phe Phe Pro Ile Pro Leu Pro Tyr Cys Trp Leu Cys Arg Ala Leu
   625                 630                 635                 640
   Ile Lys Arg Ile Gln Ala Met Ile Pro Lys Gly Ala Leu Ala Val Ala
                   645                 650                 655
   Val Ala Gln Val Cys Arg Val Val Pro Leu Val Ala Gly Gly Ile Cys
               660                 665                 670
   Gln Cys Leu Ala Glu Arg Tyr Ser Val Ile Leu Leu Asp Thr Leu Leu
           675                 680                 685
   Gly Arg Met Leu Pro Gln Leu Val Cys Arg Leu Val Leu Arg Cys Ser
       690                 695                 700
   Met
   705
<210> 7
   <211> 1761
   <212> DNA
   <213> Artificial Sequence
<220>
   <223> cDNA sequence encoding the fusion cassette consisting of Lectin - YPet - TEV - SP-B - TEV
<400> 7
   gagactgtgt ccttttcatg gaacaagttt gttccaaaac agcccaatat gattctccaa 60
   ggtgatgcca ttgttacctc atccggtaaa ctccagttga ataaggtaga tgagaacggt 120
   acaccaaaac catcctctct gggtagagca ctttactcaa ccccaattca catttgggat 180
   aaagaaacag gatctgtggc tagttttgct gcaagtttta actttacctt ttacgcacct 240
   gacactaaac gactagcaga tggtcttgct ttctttttgg ctcccattga tactaaacct 300
   caaacccatg cagggtactt aggactcttc aatgagaatg agagtggtga tcaggttgtt 360
   gctgtggagt ttgatacttt tcgaaatagc tgggatcctc caaatccaca catagggatc 420
   aatgttaatt ctataaggtc aattaagact actagttggg acttggccaa taacaaagta 480
   gctaaagtgc ttattacata tgacgcttca acctctctat tggtggcttc tctcgtctac 540
   ccttcacaaa gaacaagcaa tatactgtcc gatgtagtgg atttgaaaac ctcactgcca 600
   gaatgggtga ggataggctt ttcagcagcc actggacttg atatacccgg tgaatctcat 660
   gacgttcttt catggtcttt tgcttctaat cttccacacg cttccagcaa tattgatcct 720
   ttggatctaa caagttttgt tctgcacgaa gctattatgt ctaaaggaga agaattgttt 780
   actggtgtag tgcctatcct tgttgaattg gatggtgatg ttaatggcca caaattctca 840
   gtaagtgggg aaggtgaagg cgacgctaca tatggaaagc tcactcttaa gctgctatgt 900
   actactggaa agttgcccgt cccttggcca acattggtta caacacttgg atatggggtt 960
   cagtgttttg caagatatcc agaccacatg aagcaacatg acttcttcaa atctgctatg 1020
   ccagagggat atgtccaaga aaggacaata ttcttcaagg atgatggaaa ttacaagaca 1080
   agagctgagg tgaagtttga aggcgataca ctggttaaca gaatcgaact aaagggcatt 1140
   gatttcaagg aagatggaaa tatcttaggg cataagctgg agtataacta taactctcat 1200
   aatgtttaca taaccgcaga caagcagaaa aacgggatta aggcaaattt caagattagg 1260
   cataacatcg aggatggagg tgttcagctg gcagaccatt atcagcaaaa tacaccaata 1320
   ggtgatggcc cagtcttatt gcctgataac cattatctct cctatcagag tgctctcttt 1380
   aaggacccta acgaaaagag agatcatatg gtcttacttg agttcttgac tgcagccgga 1440
   attaccgaag ggatgaacga actttacaag tgtacacccg agaatctcta tttccaagga 1500
   ttccctatcc cactgcctta ctgctggctt tgtcgagccc ttatcaagag gattcaagca 1560
   atgatcccaa aaggtgcctt agcagtcgcc gtggcccaag tttgcagagt agtgcctctt 1620
   gttgctggcg gaatttgcca atgtcttgca gagcgttaca gcgttattct gttggatact 1680
   ctgttgggca gaatgctacc tcaattggtg tgccgtctgg tgttacgttg tagcatgccc 1740
   gaaaatcttt actttcaagg t 1761
<210> 8
   <211> 587
   <212> PRT
   <213> Artificial Sequence
<220>
   <223> polypeptide sequence of the the fusion cassette consisting of Lectin - YPet - TEV - SP-B - TEV
<400> 8
   Glu Thr Val Ser Phe Ser Trp Asn Lys Phe Val Pro Lys Gln Pro Asn
   1               5                   10                  15
   Met Ile Leu Gln Gly Asp Ala Ile Val Thr Ser Ser Gly Lys Leu Gln
               20                  25                  30
   Leu Asn Lys Val Asp Glu Asn Gly Thr Pro Lys Pro Ser Ser Leu Gly
           35                  40                  45
   Arg Ala Leu Tyr Ser Thr Pro Ile His Ile Trp Asp Lys Glu Thr Gly
       50                  55                  60
   Ser Val Ala Ser Phe Ala Ala Ser Phe Asn Phe Thr Phe Tyr Ala Pro
   65                  70                  75                  80
   Asp Thr Lys Arg Leu Ala Asp Gly Leu Ala Phe Phe Leu Ala Pro Ile
                   85                  90                  95
   Asp Thr Lys Pro Gln Thr His Ala Gly Tyr Leu Gly Leu Phe Asn Glu
               100                 105                 110
   Asn Glu Ser Gly Asp Gln Val Val Ala Val Glu Phe Asp Thr Phe Arg
           115                 120                 125
   Asn Ser Trp Asp Pro Pro Asn Pro His Ile Gly Ile Asn Val Asn Ser
       130                 135                 140
   Ile Arg Ser Ile Lys Thr Thr Ser Trp Asp Leu Ala Asn Asn Lys Val
   145                 150                 155                 160
   Ala Lys Val Leu Ile Thr Tyr Asp Ala Ser Thr Ser Leu Leu Val Ala
                   165                 170                 175
   Ser Leu Val Tyr Pro Ser Gln Arg Thr Ser Asn Ile Leu Ser Asp Val
               180                 185                 190
   Val Asp Leu Lys Thr Ser Leu Pro Glu Trp Val Arg Ile Gly Phe Ser
           195                 200                 205
   Ala Ala Thr Gly Leu Asp Ile Pro Gly Glu Ser His Asp Val Leu Ser
       210                 215                 220
   Trp Ser Phe Ala Ser Asn Leu Pro His Ala Ser Ser Asn Ile Asp Pro
   225                 230                 235                 240
   Leu Asp Leu Thr Ser Phe Val Leu His Glu Ala Ile Met Ser Lys Gly
                   245                 250                 255
   Glu Glu Leu Phe Thr Gly Val Val Pro Ile Leu Val Glu Leu Asp Gly
               260                 265                 270
   Asp Val Asn Gly His Lys Phe Ser Val Ser Gly Glu Gly Glu Gly Asp
   275 280 285
   Ala Thr Tyr Gly Lys Leu Thr Leu Lys Leu Leu Cys Thr Thr Gly Lys
   290 295 300
   Leu Pro Val Pro Trp Pro Thr Leu Val Thr Thr Leu Gly Tyr Gly Val
   305 310 315 320
   Gln Cys Phe Ala Arg Tyr Pro Asp His Met Lys Gln His Asp Phe Phe
   325 330 335
   Lys Ser Ala Met Pro Glu Gly Tyr Val Gln Glu Arg Thr Ile Phe Phe
   340 345 350
   Lys Asp Asp Gly Asn Tyr Lys Thr Arg Ala Glu Val Lys Phe Glu Gly
   355 360 365
   Asp Thr Leu Val Asn Arg Ile Glu Leu Lys Gly Ile Asp Phe Lys Glu
   370 375 380
   Asp Gly Asn Ile Leu Gly His Lys Leu Glu Tyr Asn Tyr Asn Ser His
   385 390 395 400
   Asn Val Tyr Ile Thr Ala Asp Lys Gln Lys Asn Gly Ile Lys Ala Asn
   405 410 415
   Phe Lys Ile Arg His Asn Ile Glu Asp Gly Gly Val Gln Leu Ala Asp
   420 425 430
   His Tyr Gln Gln Asn Thr Pro Ile Gly Asp Gly Pro Val Leu Leu Pro
   435 440 445
   Asp Asn His Tyr Leu Ser Tyr Gln Ser Ala Leu Phe Lys Asp Pro Asn
   450 455 460
   Glu Lys Arg Asp His Met Val Leu Leu Glu Phe Leu Thr Ala Ala Gly
   465 470 475 480
   Ile Thr Glu Gly Met Asn Glu Leu Tyr Lys Cys Thr Pro Glu Asn Leu
   485 490 495
   Tyr Phe Gln Gly Phe Pro Ile Pro Leu Pro Tyr Cys Trp Leu Cys Arg
   500 505 510
   Ala Leu Ile Lys Arg Ile Gln Ala Met Ile Pro Lys Gly Ala Leu Ala
   515 520 525
   Val Ala Val Ala Gln Val Cys Arg Val Val Pro Leu Val Ala Gly Gly
       530                 535                 540
   Ile Cys Gln Cys Leu Ala Glu Arg Tyr Ser Val Ile Leu Leu Asp Thr
   545                 550                 555                 560
   Leu Leu Gly Arg Met Leu Pro Gln Leu Val Cys Arg Leu Val Leu Arg
                   565                 570                 575
   Cys Ser Met Pro Glu Asn Leu Tyr Phe Gln Gly
               580                 585
<210> 9
   <211> 1071
   <212> DNA
   <213> Artificial Sequence
<220>
   <223> cDNA sequence encoding the fusion cassette consisting of YPet - TEV - SP-B - TEV - polyhistidine
<400> 9
   tctaaaggag aagaattgtt tactggtgta gtgcctatcc ttgttgaatt ggatggtgat      60
   gttaatggcc acaaattctc agtaagtggg gaaggtgaag gcgacgctac atatggaaag     120
   ctcactctta agctgctatg tactactgga aagttgcccg tcccttggcc aacattggtt     180
   acaacacttg gatatggggt tcagtgtttt gcaagatatc cagaccacat gaagcaacat     240
   gacttcttca aatctgctat gccagaggga tatgtccaag aaaggacaat attcttcaag     300
   gatgatggaa attacaagac aagagctgag gtgaagtttg aaggcgatac actggttaac     360
   agaatcgaac taaagggcat tgatttcaag gaagatggaa atatcttagg gcataagctg     420
   gagtataact ataactctca taatgtttac ataaccgcag acaagcagaa aaacgggatt     480
   aaggcaaatt tcaagattag gcataacatc gaggatggag gtgttcagct ggcagaccat     540
   tatcagcaaa atacaccaat aggtgatggc ccagtcttat tgcctgataa ccattatctc     600
   tcctatcaga gtgctctctt taaggaccct aacgaaaaga gagatcatat ggtcttactt     660
   gagttcttga ctgcagccgg aattaccgaa gggatgaacg aactttacaa gtgtacaccc     720
   gagaatctct attttcaagg tttccctatc ccactgcctt actgctggct ttgtcgagcc     780
   cttatcaaga ggattcaagc aatgatccca aaaggtgcct tagcagtcgc cgtggcccaa     840
   gtttgcagag tagtgcctct tgttgctggc ggaatttgcc aatgtcttgc agagcgttac     900
   agcgttattc tgttggatac tctgttgggc agaatgctac ctcaattggt gtgccgtctg     960
   gtgttacgtt gtagcatgcc cgaaaatctt tactttcaag gtggttacca acaccatcat    1020
   catcaccacc atcatcatca ccaccatcac catcatcatc accaccacca t             1071
<210> 10
   <211> 357
   <212> PRT
   <213> Artificial Sequence
<220>
   <223> polypeptide sequence of the the fusion cassette consisting of YPet - TEV - SP-B - TEV - polyhistidine
<400> 10
   Ser Lys Gly Glu Glu Leu Phe Thr Gly Val Val Pro Ile Leu Val Glu
   1               5                   10                  15
   Leu Asp Gly Asp Val Asn Gly His Lys Phe Ser Val Ser Gly Glu Gly
               20                  25                  30
   Glu Gly Asp Ala Thr Tyr Gly Lys Leu Thr Leu Lys Leu Leu Cys Thr
           35                  40                  45
   Thr Gly Lys Leu Pro Val Pro Trp Pro Thr Leu Val Thr Thr Leu Gly
       50                  55                  60
   Tyr Gly Val Gln Cys Phe Ala Arg Tyr Pro Asp His Met Lys Gln His
   65                  70                  75                  80
   Asp Phe Phe Lys Ser Ala Met Pro Glu Gly Tyr Val Gln Glu Arg Thr
                   85                  90                  95
   Ile Phe Phe Lys Asp Asp Gly Asn Tyr Lys Thr Arg Ala Glu Val Lys
               100                 105                 110
   Phe Glu Gly Asp Thr Leu Val Asn Arg Ile Glu Leu Lys Gly Ile Asp
           115                 120                 125
   Phe Lys Glu Asp Gly Asn Ile Leu Gly His Lys Leu Glu Tyr Asn Tyr
       130                 135                 140
   Asn Ser His Asn Val Tyr Ile Thr Ala Asp Lys Gln Lys Asn Gly Ile
   145                 150                 155                 160
   Lys Ala Asn Phe Lys Ile Arg His Asn Ile Glu Asp Gly Gly Val Gln
                   165                 170                 175
   Leu Ala Asp His Tyr Gln Gln Asn Thr Pro Ile Gly Asp Gly Pro Val
               180                 185                 190
   Leu Leu Pro Asp Asn His Tyr Leu Ser Tyr Gln Ser Ala Leu Phe Lys
           195                 200                 205
   Asp Pro Asn Glu Lys Arg Asp His Met Val Leu Leu Glu Phe Leu Thr
       210                 215                 220
   Ala Ala Gly Ile Thr Glu Gly Met Asn Glu Leu Tyr Lys Cys Thr Pro
   225                 230                 235                 240
   Glu Asn Leu Tyr Phe Gln Gly Phe Pro Ile Pro Leu Pro Tyr Cys Trp
                   245                 250                 255
   Leu Cys Arg Ala Leu Ile Lys Arg Ile Gln Ala Met Ile Pro Lys Gly
               260                 265                 270
   Ala Leu Ala Val Ala Val Ala Gln Val Cys Arg Val Val Pro Leu Val
           275                 280                 285
   Ala Gly Gly Ile Cys Gln Cys Leu Ala Glu Arg Tyr Ser Val Ile Leu
       290                 295                 300
   Leu Asp Thr Leu Leu Gly Arg Met Leu Pro Gln Leu Val Cys Arg Leu
   305                 310                 315                 320
   Val Leu Arg Cys Ser Met Pro Glu Asn Leu Tyr Phe Gln Gly Gly Tyr
                   325                 330                 335
   Gln His His His His His His His His His His His His His His His
               340                 345                 350
   His His His His His
           355
<210> 11
   <211> 1476
   <212> DNA
   <213> Artificial Sequence
<220>
   <223> cDNA sequence encoding the fusion cassette consisting of YPet - chymosin - SP-B - TEV - Leptin
<400> 11
   tctaaaggag aagaattgtt tactggtgta gtgcctatcc ttgttgaatt ggatggtgat      60
   gttaatggcc acaaattctc agtaagtggg gaaggtgaag gcgacgctac atatggaaag     120
   ctcactctta agctgctatg tactactgga aagttgcccg tcccttggcc aacattggtt     180
   acaacacttg gatatggggt tcagtgtttt gcaagatatc cagaccacat gaagcaacat     240
   gacttcttca aatctgctat gccagaggga tatgtccaag aaaggacaat attcttcaag     300
   gatgatggaa attacaagac aagagctgag gtgaagtttg aaggcgatac actggttaac     360
   agaatcgaac taaagggcat tgatttcaag gaagatggaa atatcttagg gcataagctg 420
   gagtataact ataactctca taatgtttac ataaccgcag acaagcagaa aaacgggatt 480
   aaggcaaatt tcaagattag gcataacatc gaggatggag gtgttcagct ggcagaccat 540
   tatcagcaaa atacaccaat aggtgatggc ccagtcttat tgcctgataa ccattatctc 600
   tcctatcaga gtgctctctt taaggaccct aacgaaaaga gagatcatat ggtcttactt 660
   gagttcttga ctgcagccgg aattaccgaa gggatgaacg aactttacaa gtgtacagag 720
   gactttctac aaaaacaaca atatggaata agctcaaaat tcttccctat cccactgcct 780
   tactgctggc tttgtcgagc ccttatcaag aggattcaag caatgatccc aaaaggtgcc 840
   ttagcagtcg ccgtggccca agtttgcaga gtagtgcctc ttgttgctgg cggaatttgc 900
   caatgtcttg cagagcgtta cagcgttatt ctgttggata ctctgttggg cagaatgcta 960
   cctcaattgg tgtgccgtct ggtgttacgt tgtagcatgc ccgaaaatct ttactttcaa 1020
   ggtggttacc aaatggctgt tcctatacag aaggttcaag atgacacaaa aactttgatt 1080
   aagactattg tgaccagaat caatgatatt tcacacactc agagtgtcag ctccaaacag 1140
   aaggtaacag gactagactt cataccagga ttgcatccaa tattgacatt atcaaaaatg 1200
   gatcaaaccc ttgcagttta tcagcaaatc ttaacctcta tgccaagtag aaatgttata 1260
   cagattagta acgatctcga gaacttaaga gacttgcttc acgttttggc attttctaag 1320
   tcttgtcatt tgccttgggc atccggtctt gagacactgg atagtctcgg aggtgtgttg 1380
   gaagcatctg ggtattcaac tgaagttgta gctctatcaa ggttgcaggg ttctcttcaa 1440
   gatatgctgt ggcagttgga tctttctcct ggttgt 1476
<210> 12
   <211> 492
   <212> PRT
   <213> Artificial Sequence
<220>
   <223> polypeptide sequence of the the fusion cassette consisting of YPet - chymosin - SP-B - TEV - Leptin
<400> 12
   Ser Lys Gly Glu Glu Leu Phe Thr Gly Val Val Pro Ile Leu Val Glu
   1               5                   10                  15
   Leu Asp Gly Asp Val Asn Gly His Lys Phe Ser Val Ser Gly Glu Gly
               20                  25                  30
   Glu Gly Asp Ala Thr Tyr Gly Lys Leu Thr Leu Lys Leu Leu Cys Thr
           35                  40                  45
   Thr Gly Lys Leu Pro Val Pro Trp Pro Thr Leu Val Thr Thr Leu Gly
       50                  55                  60
   Tyr Gly Val Gln Cys Phe Ala Arg Tyr Pro Asp His Met Lys Gln His
   65                  70                  75                  80
   Asp Phe Phe Lys Ser Ala Met Pro Glu Gly Tyr Val Gln Glu Arg Thr
                   85                  90                  95
   Ile Phe Phe Lys Asp Asp Gly Asn Tyr Lys Thr Arg Ala Glu Val Lys
               100                 105                 110
   Phe Glu Gly Asp Thr Leu Val Asn Arg Ile Glu Leu Lys Gly Ile Asp
           115                 120                 125
   Phe Lys Glu Asp Gly Asn Ile Leu Gly His Lys Leu Glu Tyr Asn Tyr
       130                 135                 140
   Asn Ser His Asn Val Tyr Ile Thr Ala Asp Lys Gln Lys Asn Gly Ile
   145                 150                 155                 160
   Lys Ala Asn Phe Lys Ile Arg His Asn Ile Glu Asp Gly Gly Val Gln
                   165                 170                 175
   Leu Ala Asp His Tyr Gln Gln Asn Thr Pro Ile Gly Asp Gly Pro Val
               180                 185                 190
   Leu Leu Pro Asp Asn His Tyr Leu Ser Tyr Gln Ser Ala Leu Phe Lys
           195                 200                 205
   Asp Pro Asn Glu Lys Arg Asp His Met Val Leu Leu Glu Phe Leu Thr
       210                 215                 220
   Ala Ala Gly Ile Thr Glu Gly Met Asn Glu Leu Tyr Lys Cys Thr Glu
   225                 230                 235                 240
   Asp Phe Leu Gln Lys Gln Gln Tyr Gly Ile Ser Ser Lys Phe Phe Pro
                   245                 250                 255
   Ile Pro Leu Pro Tyr Cys Trp Leu Cys Arg Ala Leu Ile Lys Arg Ile
               260                 265                 270
   Gln Ala Met Ile Pro Lys Gly Ala Leu Ala Val Ala Val Ala Gln Val
           275                 280                 285
   Cys Arg Val Val Pro Leu Val Ala Gly Gly Ile Cys Gln Cys Leu Ala
       290                 295                 300
   Glu Arg Tyr Ser Val Ile Leu Leu Asp Thr Leu Leu Gly Arg Met Leu
   305                 310                 315                 320
   Pro Gln Leu Val Cys Arg Leu Val Leu Arg Cys Ser Met Pro Glu Asn
                   325                 330                 335
   Leu Tyr Phe Gln Gly Gly Tyr Gln Met Ala Val Pro Ile Gln Lys Val
               340                 345                 350
   Gln Asp Asp Thr Lys Thr Leu Ile Lys Thr Ile Val Thr Arg Ile Asn
           355                 360                 365
   Asp Ile Ser His Thr Gln Ser Val Ser Ser Lys Gln Lys Val Thr Gly
       370                 375                 380
   Leu Asp Phe Ile Pro Gly Leu His Pro Ile Leu Thr Leu Ser Lys Met
   385                 390                 395                 400
   Asp Gln Thr Leu Ala Val Tyr Gln Gln Ile Leu Thr Ser Met Pro Ser
                   405                 410                 415
   Arg Asn Val Ile Gln Ile Ser Asn Asp Leu Glu Asn Leu Arg Asp Leu
               420                 425                 430
   Leu His Val Leu Ala Phe Ser Lys Ser Cys His Leu Pro Trp Ala Ser
           435                 440                 445
   Gly Leu Glu Thr Leu Asp Ser Leu Gly Gly Val Leu Glu Ala Ser Gly
       450                 455                 460
   Tyr Ser Thr Glu Val Val Ala Leu Ser Arg Leu Gln Gly Ser Leu Gln
   465                 470                 475                 480
   Asp Met Leu Trp Gln Leu Asp Leu Ser Pro Gly Cys
                   485                 490
<210> 13
   <211> 1341
   <212> DNA
   <213> Artificial Sequence
<220>
   <223> cDNA sequence encoding the fusion cassette consisting of LEA - chymosin - SP-B - TEV - Ypet
<400> 13
   caatcagcta agcagaagat ttccgatatg gcttctactg ctaaagaaaa gatggttatt      60
   tgtcaggcta aagctgacga aaaggctgaa cgagctatgg ccaggacaaa agaagaaaag     120
   gaaatagccc atcagaggag aaaggccaag gaggctgacg caaatatgga tatgcacatg 180
   gccaaggctg cacacgctga ggataaactt atggctaaac aaagtcacta tcatgtcaca 240
   gatcatgggc cacatgtttc acagcaagca tctgtgccat ccccagcacc agttatggca 300
   catggctgta cagaggactt tctacaaaaa caacaatatg gaataagctc aaaattcttc 360
   cctatcccac tgccttactg ctggctttgt cgagccctta tcaagaggat tcaagcaatg 420
   atcccaaaag gtgccttagc agtcgccgtg gcccaagttt gcagagtagt gcctcttgtt 480
   gctggcggaa tttgccaatg tcttgcagag cgttacagcg ttattctgtt ggatactctg 540
   ttgggcagaa tgctacctca attggtgtgc cgtctggtgt tacgttgtag catgcccgaa 600
   aatctttact ttcaaggtgg ttaccaaatg tctaaaggag aagaattgtt tactggtgta 660
   gtgcctatcc ttgttgaatt ggatggtgat gttaatggcc acaaattctc agtaagtggg 720
   gaaggtgaag gcgacgctac atatggaaag ctcactctta agctgctatg tactactgga 780
   aagttgcccg tcccttggcc aacattggtt acaacacttg gatatggggt tcagtgtttt 840
   gcaagatatc cagaccacat gaagcaacat gacttcttca aatctgctat gccagaggga 900
   tatgtccaag aaaggacaat attcttcaag gatgatggaa attacaagac aagagctgag 960
   gtgaagtttg aaggcgatac actggttaac agaatcgaac taaagggcat tgatttcaag 1020
   gaagatggaa atatcttagg gcataagctg gagtataact ataactctca taatgtttac 1080
   ataaccgcag acaagcagaa aaacgggatt aaggcaaatt tcaagattag gcataacatc 1140
   gaggatggag gtgttcagct ggcagaccat tatcagcaaa atacaccaat aggtgatggc 1200
   ccagtcttat tgcctgataa ccattatctc tcctatcaga gtgctctctt taaggaccct 1260
   aacgaaaaga gagatcatat ggtcttactt gagttcttga ctgcagccgg aattaccgaa 1320
   gggatgaacg aactttacaa g 1341
<210> 14
   <211> 447
   <212> PRT
   <213> Artificial Sequence
<220>
   <223> polypeptide sequence of the the fusion cassette consisting of LEA - chymosin - SP-B - TEV - Ypet
<400> 14
   Gln Ser Ala Lys Gln Lys Ile Ser Asp Met Ala Ser Thr Ala Lys Glu
   1               5                   10                  15
   Lys Met Val Ile Cys Gln Ala Lys Ala Asp Glu Lys Ala Glu Arg Ala
               20                  25                  30
   Met Ala Arg Thr Lys Glu Glu Lys Glu Ile Ala His Gln Arg Arg Lys
               35                  40                  45
   Ala Lys Glu Ala Asp Ala Asn Met Asp Met His Met Ala Lys Ala Ala
       50                  55                  60
   His Ala Glu Asp Lys Leu Met Ala Lys Gln Ser His Tyr His Val Thr
   65                  70                  75                  80
   Asp His Gly Pro His Val Ser Gln Gln Ala Ser Val Pro Ser Pro Ala
                   85                  90                  95
   Pro Val Met Ala His Gly Cys Thr Glu Asp Phe Leu Gln Lys Gln Gln
               100                 105                 110
   Tyr Gly Ile Ser Ser Lys Phe Phe Pro Ile Pro Leu Pro Tyr Cys Trp
           115                 120                 125
   Leu Cys Arg Ala Leu Ile Lys Arg Ile Gln Ala Met Ile Pro Lys Gly
       130                 135                 140
   Ala Leu Ala Val Ala Val Ala Gln Val Cys Arg Val Val Pro Leu Val
   145                 150                 155                 160
   Ala Gly Gly Ile Cys Gln Cys Leu Ala Glu Arg Tyr Ser Val Ile Leu
                   165                 170                 175
   Leu Asp Thr Leu Leu Gly Arg Met Leu Pro Gln Leu Val Cys Arg Leu
               180                 185                 190
   Val Leu Arg Cys Ser Met Pro Glu Asn Leu Tyr Phe Gln Gly Gly Tyr
           195                 200                 205
   Gln Met Ser Lys Gly Glu Glu Leu Phe Thr Gly Val Val Pro Ile Leu
       210                 215                 220
   Val Glu Leu Asp Gly Asp Val Asn Gly His Lys Phe Ser Val Ser Gly
   225                 230                 235                 240
   Glu Gly Glu Gly Asp Ala Thr Tyr Gly Lys Leu Thr Leu Lys Leu Leu
                   245                 250                 255
   Cys Thr Thr Gly Lys Leu Pro Val Pro Trp Pro Thr Leu Val Thr Thr
               260                 265                 270
   Leu Gly Tyr Gly Val Gln Cys Phe Ala Arg Tyr Pro Asp His Met Lys
           275                 280                 285
   Gln His Asp Phe Phe Lys Ser Ala Met Pro Glu Gly Tyr Val Gln Glu
       290                 295                 300
   Arg Thr Ile Phe Phe Lys Asp Asp Gly Asn Tyr Lys Thr Arg Ala Glu
   305                 310                 315                 320
   Val Lys Phe Glu Gly Asp Thr Leu Val Asn Arg Ile Glu Leu Lys Gly
                   325                 330                 335
   Ile Asp Phe Lys Glu Asp Gly Asn Ile Leu Gly His Lys Leu Glu Tyr
               340                 345                 350
   Asn Tyr Asn Ser His Asn Val Tyr Ile Thr Ala Asp Lys Gln Lys Asn
           355                 360                 365
   Gly Ile Lys Ala Asn Phe Lys Ile Arg His Asn Ile Glu Asp Gly Gly
       370                 375                 380
   Val Gln Leu Ala Asp His Tyr Gln Gln Asn Thr Pro Ile Gly Asp Gly
   385                 390                 395                 400
   Pro Val Leu Leu Pro Asp Asn His Tyr Leu Ser Tyr Gln Ser Ala Leu
                   405                 410                 415
   Phe Lys Asp Pro Asn Glu Lys Arg Asp His Met Val Leu Leu Glu Phe
               420                 425                 430
   Leu Thr Ala Ala Gly Ile Thr Glu Gly Met Asn Glu Leu Tyr Lys
           435                 440                 445
<210> 15
   <211> 1341
   <212> DNA
   <213> Artificial Sequence
<220>
   <223> cDNA sequence encoding the fusion cassette consisting of YPet - chymosin - SP-B - TEV - LEA
<400> 15
   tctaaaggag aagaattgtt tactggtgta gtgcctatcc ttgttgaatt ggatggtgat 60
   gttaatggcc acaaattctc agtaagtggg gaaggtgaag gcgacgctac atatggaaag 120
   ctcactctta agctgctatg tactactgga aagttgcccg tcccttggcc aacattggtt 180
   acaacacttg gatatggggt tcagtgtttt gcaagatatc cagaccacat gaagcaacat 240
   gacttcttca aatctgctat gccagaggga tatgtccaag aaaggacaat attcttcaag 300
   gatgatggaa attacaagac aagagctgag gtgaagtttg aaggcgatac actggttaac 360
   agaatcgaac taaagggcat tgatttcaag gaagatggaa atatcttagg gcataagctg 420
   gagtataact ataactctca taatgtttac ataaccgcag acaagcagaa aaacgggatt 480
   aaggcaaatt tcaagattag gcataacatc gaggatggag gtgttcagct ggcagaccat 540
   tatcagcaaa atacaccaat aggtgatggc ccagtcttat tgcctgataa ccattatctc 600
   tcctatcaga gtgctctctt taaggaccct aacgaaaaga gagatcatat ggtcttactt 660
   gagttcttga ctgcagccgg aattaccgaa gggatgaacg aactttacaa gtgtacagag 720
   gactttctac aaaaacaaca atatggaata agctcaaaat tcttccctat cccactgcct 780
   tactgctggc tttgtcgagc ccttatcaag aggattcaag caatgatccc aaaaggtgcc 840
   ttagcagtcg ccgtggccca agtttgcaga gtagtgcctc ttgttgctgg cggaatttgc 900
   caatgtcttg cagagcgtta cagcgttatt ctgttggata ctctgttggg cagaatgcta 960
   cctcaattgg tgtgccgtct ggtgttacgt tgtagcatgc ccgaaaatct ttactttcaa 1020
   ggtggttacc aaatgcaatc agctaagcag aagatttccg atatggcttc tactgctaaa 1080
   gaaaagatgg ttatttgtca ggctaaagct gacgaaaagg ctgaacgagc tatggccagg 1140
   acaaaagaag aaaaggaaat agcccatcag aggagaaagg ccaaggaggc tgacgcaaat 1200
   atggatatgc acatggccaa ggctgcacac gctgaggata aacttatggc taaacaaagt 1260
   cactatcatg tcacagatca tgggccacat gtttcacagc aagcatctgt gccatcccca 1320
   gcaccagtta tggcacatgg c 1341
<210> 16
   <211> 447
   <212> PRT
   <213> Artificial Sequence
<220>
   <223> polypeptide sequence of the the fusion cassette consisting of YPet - chymosin - SP-B - TEV - LEA
<400> 16
   Ser Lys Gly Glu Glu Leu Phe Thr Gly Val Val Pro Ile Leu Val Glu
   1               5                   10                  15
   Leu Asp Gly Asp Val Asn Gly His Lys Phe Ser Val Ser Gly Glu Gly
               20                  25                  30
   Glu Gly Asp Ala Thr Tyr Gly Lys Leu Thr Leu Lys Leu Leu Cys Thr
           35                  40                  45
   Thr Gly Lys Leu Pro Val Pro Trp Pro Thr Leu Val Thr Thr Leu Gly
       50                  55                  60
   Tyr Gly Val Gln Cys Phe Ala Arg Tyr Pro Asp His Met Lys Gln His
   65                  70                  75                  80
   Asp Phe Phe Lys Ser Ala Met Pro Glu Gly Tyr Val Gln Glu Arg Thr
                   85                  90                  95
   Ile Phe Phe Lys Asp Asp Gly Asn Tyr Lys Thr Arg Ala Glu Val Lys
               100                 105                 110
   Phe Glu Gly Asp Thr Leu Val Asn Arg Ile Glu Leu Lys Gly Ile Asp
           115                 120                 125
   Phe Lys Glu Asp Gly Asn Ile Leu Gly His Lys Leu Glu Tyr Asn Tyr
       130                 135                 140
   Asn Ser His Asn Val Tyr Ile Thr Ala Asp Lys Gln Lys Asn Gly Ile
   145                 150                 155                 160
   Lys Ala Asn Phe Lys Ile Arg His Asn Ile Glu Asp Gly Gly Val Gln
                   165                 170                 175
   Leu Ala Asp His Tyr Gln Gln Asn Thr Pro Ile Gly Asp Gly Pro Val
               180                 185                 190
   Leu Leu Pro Asp Asn His Tyr Leu Ser Tyr Gln Ser Ala Leu Phe Lys
           195                 200                 205
   Asp Pro Asn Glu Lys Arg Asp His Met Val Leu Leu Glu Phe Leu Thr
       210                 215                 220
   Ala Ala Gly Ile Thr Glu Gly Met Asn Glu Leu Tyr Lys Cys Thr Glu
   225                 230                 235                 240
   Asp Phe Leu Gln Lys Gln Gln Tyr Gly Ile Ser Ser Lys Phe Phe Pro
                   245                 250                 255
   Ile Pro Leu Pro Tyr Cys Trp Leu Cys Arg Ala Leu Ile Lys Arg Ile
               260                 265                 270
   Gln Ala Met Ile Pro Lys Gly Ala Leu Ala Val Ala Val Ala Gln Val
           275                 280                 285
   Cys Arg Val Val Pro Leu Val Ala Gly Gly Ile Cys Gln Cys Leu Ala
       290                 295                 300
   Glu Arg Tyr Ser Val Ile Leu Leu Asp Thr Leu Leu Gly Arg Met Leu
   305                 310                 315                 320
   Pro Gln Leu Val Cys Arg Leu Val Leu Arg Cys Ser Met Pro Glu Asn
   325 330 335
   Leu Tyr Phe Gln Gly Gly Tyr Gln Met Gln Ser Ala Lys Gln Lys Ile
   340 345 350
   Ser Asp Met Ala Ser Thr Ala Lys Glu Lys Met Val Ile Cys Gln Ala
   355 360 365
   Lys Ala Asp Glu Lys Ala Glu Arg Ala Met Ala Arg Thr Lys Glu Glu
   370 375 380
   Lys Glu Ile Ala His Gln Arg Arg Lys Ala Lys Glu Ala Asp Ala Asn
   385 390 395 400
   Met Asp Met His Met Ala Lys Ala Ala His Ala Glu Asp Lys Leu Met
   405 410 415
   Ala Lys Gln Ser His Tyr His Val Thr Asp His Gly Pro His Val Ser
   420 425 430
   Gln Gln Ala Ser Val Pro Ser Pro Ala Pro Val Met Ala His Gly
   435 440 445
<210> 17
   <211> 93
   <212> DNA
   <213> Nicotiana tabacum
<400> 17
   atggcaaact ccatgcgctt ctttgcaact gtgttactta tagcattgct tgtcacggct 60
   accgagatgg gaccaatgac aattgcagag gca 93
<210> 18
   <211> 31
   <212> PRT
   <213> Nicotiana tabacum
<400> 18
   Met Ala Asn Ser Met Arg Phe Phe Ala Thr Val Leu Leu Ile Ala Leu
   1 5 10 15
   Leu Val Thr Ala Thr Glu Met Gly Pro Met Thr Ile Ala Glu Ala
   20 25 30
<210> 19
   <211> 100
   <212> DNA
   <213> Actinia equina
<400> 19
   atgtctctta gccagaacca ggccaagttt tccaagggat tcgtcgtgat gatttgggta 60
   ctattcattg cttgtgctat cacttcaact gaagctagtc 100
<210> 20
   <211> 33
   <212> PRT
   <213> Actinia equina
<400> 20
   Met Ser Leu Ser Gln Asn Gln Ala Lys Phe Ser Lys Gly Phe Val Val
   1 5 10 15
   Met Ile Trp Val Leu Phe Ile Ala Cys Ala Ile Thr Ser Thr Glu Ala
   20 25 30
   Ser
<210> 21
   <211> 21
   <212> DNA
   <213> Artificial Sequence
<220>
   <223> preproSP-B_frw primer sequence
<400> 21
   atggctgagt cacacctgct g 21
<210> 22
   <211> 23
   <212> DNA
   <213> Artificial Sequence
<220>
   <223> preproSP-B_rev primer sequence
<400> 22
   tcaaaggtcg gggctgtgga tac 23
<210> 23
   <211> 31
   <212> DNA
   <213> Artificial Sequence
<220>
   <223> preproSP-B27_frw primer sequence
<400> 23
   ccccgaattc atggctgagt cacacctgct g 31
<210> 24
   <211> 87
   <212> DNA
   <213> Artificial Sequence
<220>
   <223> preproSP-B27_rev primer sequence
<400> 24
   ggggaagctt tcaaagctcg tccttctcgc tgtgatggtg gtggtgatgt ttgtcatcgt 60
   catcaaggtc cgggctgtgg atacact 87
<210> 25
   <211> 28
   <212> DNA
   <213> Artificial Sequence
<220>
   <223> mpSP-B_frw primer sequence
<400> 25
   ggctcgagtt ccccattcct ctccccta 28
<210> 26
   <211> 29
   <212> DNA
   <213> Artificial Sequence
<220>
   <223> mpSP-B_rev primer sequence
<400> 26
   ggcagatctc atggagcacc ggaggacga 29
<210> 27
   <211> 32
   <212> DNA
   <213> Artificial Sequence
<220>
   <223> NtSP-FRW primer sequence
<400> 27
   ggctcgagat ggcaaactcc atgcgcttct tt 32
<210> 28
   <211> 28
   <212> DNA
   <213> Artificial Sequence
<220>
   <223> NtSP-REV primer sequence
<400> 28
   ccaagctttg cctctgcaat tgtcattg 28
<210> 29
   <211> 28
   <212> DNA
   <213> Artificial Sequence
<220>
   <223> NtSP27-FRW primer sequence
<400> 29
   ccaagcttca tcatcatcat catcacag 28
<210> 30
   <211> 29
   <212> DNA
   <213> Artificial Sequence
<220>
   <223> NtSP27-REV
<400> 30
   tctagatcac atggagcacc ggaggacga 29